# NEUE EUROPÄISCHE PATENTSCHRIFT

(11) **EP 1 408 034 B2**
(45) Veröffentlichungstag und Bekanntmachung des Hinweises auf die Entscheidung über den Einspruch: **25.05.2011**
(45) Hinweis auf die Patenterteilung: 08.08.2007
(21) Anmeldenummer: 03028047.3
(22) Anmeldetag: 22.12.2000
(51) Int. Cl.: C07D 215/56, A61K 31/47

(54) **Verwendung von Chinolon- und Chinolizinon-Derivaten als Chemotherapeutika**
Use of Quinolone and Quinolizinone derivatives as chemotherapeutic agents
Utilisation des dérivés des quinolones et quinolizinones comme agents chemothérapeutiques

(30) Priorität: 22.12.1999 DE 19962470
(43) Veröffentlichungstag der Anmeldung: 14.04.2004
(62) Teilanmeldung aus: 00985241.9
(73) Patentinhaber: Bayer Innovation GmbH, 40225 Düsseldorf (DE)
(72) Erfinder: Schulz, Hans-Herrmann, 51067 Köln (DE); Schlimbach, Günther Dr., 51469 Bergisch Gladbach (DE)
(74) Vertreter: Kutzenberger, Helga

(56) Entgegenhaltungen:
- WO-A-00/18404
- WO-A-99/07706
- WO-A1-97/44034
- JP-A- 02 275 820
- US-A- 4 399 134
- US-A- 5 478 829
- US-A- 5 693 337
- US-A- 6 017 912
- S. T. BOYCE ET. AL.: "Cytotoxicity Testing of Topical Antimicrobial Agents on Human Keratinocytes and Fibroblasts for Cultured Skin Grafts." JOURNAL OF BURN CARE AND REHABILITATION, Bd. 16, März 1995 (1995-03), Seiten 97-103, XP001036942
- MORITA S. ET AL CHEM. PHARM. BULL. Bd. 38, Nr. 7, 1990, Seiten 2027 - 2029
- MASAYA TAKEI ET AL ANTIMICROBIAL AGENTS AND CHEMOTHERAPY Bd. 45, Nr. 12, Dezember 2001, Seiten 3544 - 3547
- WEINBERG ET AL DERMATOL. THER. Bd. 16, Nr. 3, 2003, Seiten 195 - 205

## Beschreibung

Die vorliegende Erfindung betrifft die Verwendung von Chemotherapeutika zur Herstellung eines Arzneimittels zur topischen und/oder lokalen Behandlung von bakteriell verursachten Erkrankungen und/oder zur Prophylaxe bei Mensch oder Tier.

Bakterien können die Ursache einer Vielzahl von Erkrankungen sein und zudem die Wundheilung beeinträchtigen. Im Mundbereich wird beispielsweise die Zahnfäule (Karies) durch mundspezifische Keime hervorgerufen. Mundbakterien setzen Kohlenhydrate der Nahrung zu Säuren um, die den Zahnschmelz (Enamelum) und Dentin (Dentinum) auflösen können. Ist die Schmelzoberfläche eingebrochen, dringen die Bakterien weiter in das darunter liegende Dentin vor. In den radiär verlaufenden Dentinkanälchen liegen Fortsätze der Pulpa, so dass es im weiteren Verlauf zu einer partiellen oder totalen Infektion und damit Entzündung der Pulpa kommt. Eine Pulpaentzündung bewirkt einen verstärkten Blutandrang. Da die Pulpa in der starren Pulpenkammer liegt, kann sie sich nicht ausdehnen, es kommt zu Schmerzen. Erfolgt keine Behandlung, ist die weitere Folge ein Absterben des Pulpengewebes (Nekrose) und ein bakterieller Zerfall (Gangrän). Werden die gangränösen Massen nicht entfernt, sind Entzündungen ausserhalb der Wurzelspitze die Folge. Granulome, Zysten, Fistelbildung oder Abzesse können sich entwickeln. Durch Gasentwicklung treten auch jetzt verstärkt Schmerzen auf.

Auch an der Entzündung des Zahnhalteapparates (Parodontium) sind Bakterien beteiligt. Der Zahnhalteapparat besteht aus dem Zahnfleisch, dem Ringband, der Wurzelhaut und und den dazwischen liegenden Sharpey'schen Fasern. Eine Entzündung, die Parodontitis, kann einzelne Bereiche oder auch das gesamte Parodontium umfassen. Die Parodontitis ebenso wie Karies wird hervorgerufen durch Zahnbelag (Plaque) an den Rändern des Zahnfleisches, der sich durch Kalkeinlagerungen zum Zahnstein verfestigt. In der Plaque lebende Bakterien bilden Stoffwechselprodukte, die eine Parodontopathie verursachen. Das Zahnfleisch bildet sich allmählich zurück, Wurzelhaut und Alveolarknochen beginnen sich aufzulösen. Im umgebenden Gewebe bilden sich Infektionsherde. Die Folgen sind die weitere Zerstörung der Knochen und eine Lockerung der Zähne. Schließlich fallen die Zähne aus. Schäden im Mundbereich können auch zu Erkrankungen anderer Körperorgane führen.

Zur Behandlung der Caries profunda werden sowohl bei freiliegender Pulpa als auch bei noch geschlossener Dentindecke Calciumhydroxid-Präparate oder Zinkoxid-Nelkenöl (Eugenol) eingesetzt. Eine infizierte und nekrotisierte Pulpa wird möglichst vollständig entfernt und das Pulpenkavum mit geeigneten Wurzelfüllmaterialien ausgefüllt. Liegt eine Gangrän der Pulpa vor, so ist ein Erfolg nur dann zu erwarten, wenn es gelingt, das verseuchte Wurzelkanalsystem und das kanalnahe Dentin zu desinfizieren. Das Hauptproblem besteht in der Unzugänglichkeit des bakteriell infizierten apikalen Deltas der Wurzelkanäle, das nur schwer zu reinigen ist, des weiteren in dem infizierten Kanalwandsystem, das mit erheblichem instrumentellen Aufwand entfernt werden muss. Eine Gangränbehandlung ist deshalb in der Regel eine Kompromisslösung und wird von einigen Schulen ganz abgelehnt. Als Kontraindikation für eine Gangränbehandlung gelten grundsätzlich mehrwurzlige Zähne und röntgenologisch sichtbare apikale Prozesse. In diesen Fällen ist die Extraktion des Zahnes indiziert. Parodontopathien werden durch die Entfernung subgingivaler Konkremente behandelt. Darüber hinaus werden auch Arzneimittel wie Kaustika, Antiphlogistika oder Vitamine für die Behandlung eingesetzt.

Boyce et al. (J Burn Care & Rehabilitation, V16(2), p:97 103) offenbart die Verwendung von antimikrobiellen Substanzen zur Behandlung von Brandwundinfektionen.

US 5,693,337 offenbart eine lagerungsstabile Lipidemulsion; die Zitronensäure und Methionin, Phenylalanin, Serin oder Histidin als Stabilisatoren aufweist. Die Lipidemulsion gemäß US 5,693,337 kann auch Antibiotika enthalten, wie beispielsweise Levofloxacn oder Ofloxacin.

In US 5,478,829 wird eine pharmazeutische Zusammensetzung beschrieben, die Sparfloxacin enthält und zur parenteralen Verabreichung geeignet ist.

WO 99/07706 offenbart Quinolon-Carbonsäurederivate mit antibakteriellen Eigenschaften und geringer Zytotoxizität.

WO 00/18404 offenbart ophthalmische, otische und nasale pharmazeutische Zusammensetzungen, die Antibiotika enthalten, wie beispielsweise Moxifloxacin.

Für die Behandlung von Infektionen im Mundbereich setzt man antibakteriell wirksame Antibiotika und Chemotherapeutika ein, die jedoch nur nach kritischer Indikationsstellung verabreicht werden sollten. Die verwendeten Antibiotika wie Penicillin G oder Oralpenicilline sind die Mittel der ersten Wahl. Alternativ kommen Erythromycin, Lincomycin, Clindamycin, eventuell auch Sulfonamide in Betracht. Bei Mischinfektionen mit gramnegativen Keimen kommen Breitspektrum-Penicilline wie Ampicillin als Mittel der ersten Wahl in Betracht, die gegebenenfalls durch Tetracycline ersetzt werden können. Die Antibiotika und Chemotherapeutika werden immer systemisch gegeben. Es gilt die herrschende Lehrmeinung, dass zur systemischen Chemotherapie verwendete Mittel nicht als Lokalantibiotika eingesetzt werden sollen.

Den heutigen Einsatz von antimikrobiellen Substanzen in der Zahnmedizin beschreiben B. M. Owens und N. J. Schuman (Journal of Clinical Pediatric Dentistry, 1994 (Winter), 18, 129-134; zitiert in Medline, AN 94331337). Demnach werden zwei Kategorien von antimikrobiellen Wirkstoffen unterschieden, die natürlich vorkommenden Substanzen aus Pilzen (Penicilline und Cephalosporine), Bakterien und Actinomyceten (Aminoglycoside) und ihre Abkömmlinge, die Antibiotika genannt werden, sowie die Verbindungen synthetischen Ursprungs (Sulfanilamide und Chinolone), die Chemotherapeutika genannt werden. Die Gruppe der Antibiotika mit Bedeutung für die Zahnmedizin sind die Penicilline, Cephalosporine und Aminoglycoside sowie Erythromycin. Wegen ihrer guten Wirksamkeit, der niedrigen Kosten und der einfachen Anwendung sind diese Antibiotika die Mittel der Wahl für viele, wenn nicht sogar für die meisten odontogenen Infektionen. Die Substanzen synthetischen Ursprungs haben einen geringeren Wert für die Zahnmedizin. Es gilt die herrschende Lehrmeinung, dass sie häufig durch hohe Kosten, einen Mangel an Wirksamkeit und Toxizität für den Patienten gekennzeichnet sind.

In In-vitro-Studien wurde die antimikrobielle Empfindlichkeit von Keimen, die eine progressive Parodontitis oder odontogene Abzesse hervorrufen, von S. Eick et al. (Int. J. Antimicrob. Agents, 1999, 12, 41-46) gegenüber modernen Antibiotika und Chemotherapeutika, nämlich Penicillin, Amoxicillin, Cefoxitin, Clindamycin, Doxycyclin, Metronidazol und Ciprofloxacin untersucht. Als Ergebnis wurde Clindamycin für antimikrobielle Behandlungen empfohlen.

Zur Behandlung von Patienten wurden Antibiotika in der Zahnmedizin sowohl systemisch als auch topisch eingesetzt. U. Wahlmann et al. (Int. J. Antimicrob. Agents, 1999, 12, 253-256) berichteten über die Effekte der systemischen Gabe von Cefuroxim zehn Minuten vor der Extraktion von Zähnen. Die mit Cefuroxim behandelte Gruppe hatte eine geringere Häufigkeit an Bakteriämien als die nicht behandelte.

K. Kosowska und P. B. Heczko (Med. Dosw. Mikrobiol., 1977, 29, 101-106; zitiert in Chemical Abstracts, CA 88:69315) berichteten über den topischen Einsatz von verschiedenen Antibiotika. Sie behandelten infizierte Zähne mit verschiedenen Antibiotika, nämlich Erythromycin, Neomycin, Chloramphenicol, Colistin, Nystatin oder Dexamethason. In etwa 55 % der Fälle wurde die aerobe Flora in den Wurzeikanälen eliminiert. Durch die Behandlung wurde jedoch die antibiotische Resistenz der Mikroorganismen, die nach Behandlung aus den Wurzelkanälen isoliert worden waren, erhöht. Die Resistenz von Staphylococcus epidermis gegenüber Erythromycin oder Methicillin wurde um das dreifache bzw. zweifache erhöht. Die meisten Staphylococcus aureus Stämme waren resistent gegenüber Erythromycin, Chloramphenicol und Penicillin.

Die Autoren der genannten Quellen kommen übereinstimmend zu dem Ergebnis, dass der topische Einsatz von Antibiotika und Chemotherapeutika, die als Lutschtabletten, Halstabletten, Pastillen, Styli, Kegel, Puder oder Salben verabreicht werden, wegen der häufig auftretenden Resistenzsteigerungen der Erreger und der hohen Sensibilisierungsquote nur eine sehr geringe Bedeutung hat. Auch in Wunden mit ausreichendem Abfluss nach außen ist der therapeutische Nutzen im allgemeinen gering. Die verwendeten Substanzen werden kaum oder gar nicht resorbiert, so dass auch keine tiefer in das Gewebe gehende Wirkung zu erwarten ist.

Demnach besteht nach dem heutigen Stand der Technik in der humanen und tierärztlichen Zahnmedizin ein hohes Bedürfnis nach Mitteln, die eine hohe Aktivität gegen die im Mund-, Zahn- und Kieferbereich oder in oralen Wunden auftretenden Keime aufweisen, die schnell und bakterizid wirksam sind, die lokal gut verträglich sind, die eine geringe Neigung zur Erzeugung antibiotischer Resistenz hervorrufen, die topisch und/oder lokal angewendet werden können und somit einfach zu verabreichen sind, die bei topischer/lokaler Gabe eine minimale systemische Belastung des Organismus bewirken, die eine gute Gewebegängigkeit aufweisen, und deren Anwendung den Erhalt der geschädigten Zähne gewährleistet.

Bakterien sind auch bei der Wundversorgung von Bedeutung. Wunden sind Defekte des Gewebes an der Körperoberfläche und können durch Verletzungen, Operationen, Infektionen oder pathophysiologische Prozesse hervorgerufen werden. Wunden sind unter anderem wegen möglicher Infektionen aufgrund eindringender krankheitserregender Bakterien gefährlich. Die bakterielle Besiedelung der Wunde kann den Prozess der Wundheilung verlangsamen oder verhindern oder zu weiteren Komplikationen wie Lymphangitis, Sepsis oder chronischen Infektionen führen.

Infizierte Wunden müssen eine antimikrobielle Behandlung zur Bekämpfung pathogener Keime erfahren. Außerdem muß die Wunde - ähnlich wie bei nekrotischen Wunden - einer Reinigung von Fremdkörpern und Zelltrümmern unterzogen werden, damit sie in die nächste Heilungsphase eintreten kann. Die Behandlung einer infizierten Wunde besteht üblicherweise in einem kombinierten systemischen und lokalen Ansatz. Dabei werden gegebenenfalls Antibiotika eingesetzt und ein geeigneter Verband angelegt, der selbst antibakteriell wirken kann. In vielen Fällen lassen sich Wundinfektionen durch Gabe von systemisch wirksamen Antibiotika erfolgreich behandeln. Die systemische Gabe eines Antibiotikums führt jedoch zwangsläufig zu einer Belastung des gesamten Organismus. Dies kann in vielen Fällen kontraindiziert sein, beispielsweise bedingt durch die klinische Situation des Patienten, bei Grunderkrankungen des Patienten oder bei Vorliegen eines Allergisierungspotentials. In solchen Fällen ist eine topische und/oder lokale Applikation des Antibiotikums von Vorteil, was dadurch auch in einer höheren lokalen Gewebekonzentration vorliegen kann. Auch andere Faktoren wie bei bestimmten Krankenhausepidemiologien oder ökonomische Aspekte wie notwendige Aufwandmengen, Medikamentenpreise, Kosten durch Nebenwirkungen können für topische/lokale Applikationen sprechen. Der Einsatz topischer Antibiotika wird jedoch nicht generell empfohlen, da dies zu allergischen Reaktionen oder zur Ausbildung von antibiotikaresistenten Arten von Bakterien führen kann. Deshalb wird es als besonders wichtig angesehen, den Einsatz topischer Antibiotika zu limitieren.

Eine antibiotische Lokalbehandlung wird heute nur bei oberflächlichen Hautinfektionen angewandt, weil das Antibiotikum direkt auf die Erreger einwirken kann. Eine örtliche Anwendung bei tiefen Hautinfektionen ist erfolglos, weil die Antibiotika nicht durch die intakte Haut penetrieren. Heute werden bevorzugt drei Gruppen von Lokalantibiotika verwendet. Dies sind die Polypeptide wie Bacitracin, Tyrothricin, Colistin und Polymxyin B oder Aminoglycoside wie Neomycin, Kanamycin und Paromycin oder Mupirocin. Jedoch sollten Lokalantibiotika bei vorhandener lokaler bzw. systemischer Toxizität und der Gefahr einer sekundären Resistenzentwicklung der Bakterien nur mit großer Zurückhaltung angewendet werden. Bei den Lokaltherapien mit Gyrase-Hemmem sind neben konventionellen Applikationsformen wie Augentropfen, Ohrentropfen, Instillationslösungen, Puder und Wundsalben die Inkorporation in Kunststoffmaterialien als klinische Anwendungsmöglichkeiten vorgeschlagen worden (W. Stille, Fortschritte der antimikrobiellen und antineoplastischen Chemotherapie, Band 6-10, 1987, S. 1575 -1583).

Demnach besteht nach dem heutigen Stand der Technik auch in der Human- und Tiermedizin ein Bedürfnis nach topisch und/oder lokal anwendbaren antibiotisch wirksamen Mitteln, die eine hohe Aktivität gegen die in Wunden auftretenden Keime aufweisen, die schnell und bakterizid wirksam sind, die lokal gut verträglich sind, die eine geringe Neigung zur Erzeugung antibiotischer Resistenz hervorrufen, die bei topischer und/oder lokaler Anwendung einfach zu verabreichen sind, die bei topischer/lokaler Gabe eine minimale systemische Belastung des Organismus bewirken, die eine gute Gewebegängigkeit aufweisen, die auch zur Behandlung von tiefen Infektionen geeignet sind und die darüber hinaus den Prozess der Wundheilung beschleunigen.

Es wurde nun überraschenderweise gefunden, dass bestimmte Chemotherapeutika bei lokaler und/oder topischer Anwendung in vielfältiger Weise einen überaus positiven Effekt auf die Wundheilung haben. Arzneimittel, die diese Chemotherapeutika enthalten, können erfolgreich eingesetzt werden gegen Keime, die im Weichgewebe und/oder im Hartgewebe angetroffen werden und dort zu Entzündungen führen. Es wurde überraschenderweise gefunden, dass die Verwendung dieser Chemotherapeutika in der Human- und Tiermedizin in vielfältiger Weise einen positiven Effekt bei der Behandlung und der Prophylaxe von Wunden haben. Dies wurde bei der Behandlung von verschiedenen Wundformen gezeigt, beispielsweise bei chirurgischen Infektionen wie postoperativen oder posttraumatischen Wundinfektionen, bei der periooperativen Prophylaxe, bei infizierten Verbrennungen, bei Handinfektionen, bei der postoperativen Sepsis, bei infizierten Ulzera und Gangränen, bei Hautinfektionen wie akuten und chronischen bakteriellen Hautinfektionen, sekundär infizierten Dermatosen oder Akne und Rosazea. Diese Aufzählungen sind beispielhaft und bedeuten keine Einschränkung auf die genannten Bereiche.

Diese Erkrankungen lassen sich erfindungsgemäß durch lokale und/oder topische Applikation von Chemotherapeutika behandeln.

Chemotherapeutika im erfindungsgemäßen Sinne sind Derivate der Chinoloncarbonsäure oder Naphthyridoncarbonsäure der allgemeinen Formel (I) 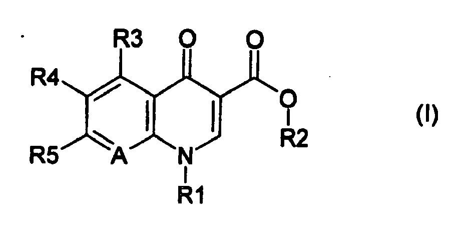 in welcher:
- A: für CH, CCl, CBr, C-CH₃, C-CN, C-OCH₃, C-OCHF₂ oder N steht,
- R1: für Cycloalkyl, Bicycloalkyl, oder 2-Fluorcyclopropyl, steht,
- R2: für Wasserstoff, Methyl oder Ethyl steht,
- R3: für Wasserstoff, Halogen, Methyl, Amino oder NH-NH₂ steht,
- R4: für Wasserstoff, Halogen oder Amino steht, und
- R5: für einen gegebenenfalls einfach oder mehrfach substituierten gesättigten oder, wenigstens eine Doppelbindung aufweisenden mono-, bi- oder tricyclischen Alicyclus mit gegebenenfalls wenigstens einem Heteroatom im Ringsystem oder einen gegebenenfalls wenigstens ein Heteroatom aufweisenden aromatischen Mono-, Bi- oder Tricyclus steht,
und/oder ihre entsprechende Hydrate und/oder ihre entsprechenden physiologisch verträglichen Säureadditionssalze und/oder ggf. ihre entsprechenden physiologisch verträglichen Salze der zugrundeliegenden Carbonsäuren, worin R2 für H steht und/oder entsprechende Enantiomere und/oder entsprechende Diastereomere und/oder entsprechende Racemate und/oder entsprechende Gemische aus wenigstens zwei der vorstehend genannten Verbindungen.

Diese Verbindungen haben bei topischer und/oder lokaler Gabe eine förderliche Wirkung bei der Behandlung von Wundformen bei Mensch und Tier bei chirurgischen Infektionen wie postoperativen oder posttraumatischen Wundinfektionen, bei der periooperativen Prophylaxe, bei infizierten Verbrennungen, bei Handinfektionen, bei der postoperativen Sepsis, bei infizierten Ulzera und Gangränen, bei akuten und chronischen bakteriellen Hautinfektionen, sekundär infizierten Dermatosen oder Akne und Rosazea sowie allgemein zur Beschleunigung der Wundheilung bei Mensch und Tier.

Bevorzugt wird wenigstens eine dieser Verbindungen zur Herstellung eines pharmazeutischen Erzeugnisses, insbesondere Arzneimittels, zur topischen und/oder lokalen Behandlung von bakteriell verursachten Erkrankungen oder zur Beschleunigung der Wundheilung verwendet.

Bevorzugt ist die Verwendung der Verbindungen der Formel (I), in welcher
- A: für CH, CCl, CBr, C-CH₃, C-CN, C-OCH₃, C-OCHF₂ oder N steht,
- R₁: für Cyclopropyl, Bicyclo(1.1.1)pent-1-yl, oder 2-Fluorcyclopropyl, steht,
- R2: für Wasserstoff, Methyl oder Ethyl steht,
- R3: für Wasserstoff, F, Cl, Br, Methyl, Amino oder NH-NH₂ steht,
- R4: für Wasserstoff, F oder Amino steht,
- R5: für gegebenenfalls einfach oder mehrfach substituiertes Cyclopropyl, Azetidinyl, Pyrrolidinyl, Piperidinyl, Piperazinyl, Morpholinyl, Phenyl, Pyrrolyl, Pyridyl oder Imidazolyl steht, wobei gegebenenfalls auch wenigstens zwei Substituenten miteinander verknüpft sein können

Besonders bevorzugt ist die Verwendung der Verbindungen der Formel (I), in welcher
- A: für CH, CCl, C-CN, C-OCH₃ oder N steht,
- R1: für Cyclopropyl oder, 2-Fluorcyclopropyl, steht,
- R2: für Wasserstoff steht,
- R3: für Wasserstoff oder Amino steht,
- R4: für Wasserstoff oder F steht,
- R5: für gegebenenfalls einfach oder mehrfach substituiertes Pyrrolidinyl, Piperidinyl, Piperazinyl oder Morpholinyl steht, wobei gegebenenfalls auch wenigstens zwei Substituenten miteinander verknüpft sein können.

Ganz besonders bevorzugt ist die Verwendung der Verbindungen der Formel (I), in welcher
- A: für CH, CCl, C-OCH₃ oder N steht,
- R1: für Cyclopropyl steht,
- R2: für Wasserstoff steht,
- R3: für Wasserstoff oder Amino steht,
- R4: für Wasserstoff oder F steht,
- R5: für gegebenenfalls mit Amino, Methyl, Aminomethyl und/oder Methoxyimino substituiertes Pyrrolidinyl, Piperidinyl, Piperazinyl, Morpholinyl, 3-Azabicyclo(3.1.0)hexyl oder für Piperidinopyrrolidinyl steht.

Unter bi- oder tricyclischen Alicyclen bzw. aromatischen Bi- oder Tricyclen gemäß R5 der allgemeinen Formel (I) werden auch kondensierte Ringsysteme verstanden, die gegebenenfalls wenigstens eine Doppelbindung und/oder wenigstens ein Heteroatom im Ringsystem aufweisen können.

Beispiele für Reste R5 in den Verbindungen der Formel (I) sind 1-Aminocyclopropyl, 3-Hydroxy-azetidin-1-yl, 3-Amino-azetidin-1-yl, 3-Methylamino-azetidin-1-yl, 3-Amino-2-methyl-azetidin-1-yl, 3-Amino-3-methylazetidin-1-yl, 3-Amino-2,3-dimethyl-azetidin-1-yl, 3-Amino-pyrrolidin-1-yl, 3-(2-amino-1-oxopropyl)-amino-1-pyrrolidin-1-yl, 3-Norvalyl-norvalyl-amino-1-pyrrolidin-1-yl, 3-Amino-3-fluormethyl-pyrrolidin-1-yl, 3-Amino-4-methyl-pyrrolidin-1-yl, 3-Amino-4-fluormethyl-pyrrolidin-1-yl, 4-Amino-2-methyl-pyrrolidin-1-yl, 4-Amino-3,3-dimethyl-1-pyrrolidin-1-yl, 3-Amino-3-phenyl-pyrrolidin-1-yl, 3-Amino-4-cyclopropyl-pyrrolidin-1-yl, 3-Aminomethyl-pyrrolidin-1-yl, 3-Ethylaminomethylpyrrolidin-1-yl, 3-(1-Aminoethyl)-1-pyrrolidin-1-yl, 3-(1-Amino-1-methyl-ethyl)-pyrrolidin-1-yl, 3-Aminomethyl-3-methyl-pyyrolidin-1-yl, 3-Aminomethyl-3-fluormethyl-pyrrolidin-1-yl, 3-Aminomethyl-4-methyl-pyyrolidin-1-yl, 3-Aminomethyl-4-trifluormethyl-pyrrolidin-1-yl, 3-Aminomethyl-4-chlor-pyrrolidin-1-yl, 3-Aminomethyl-4-methoxyimino-pyrrolidin-1-yl, 3-(2-Methyl-1H-imidazol-1-yl)-pyrrolidin-1-yl, 3-(4-Methyl-1,2,3-triazo-1-yl)-pyrrolidin-1-yl, 3-Methylaminopiperidin-1-yl, 4-Hydroxy-piperidin-1-yl, 4-Hydroxyimino-piperidin-1-yl, Piperazin-1-yl, 3-Methyl-1-piperazinyl, 4-Methyl-piperazin-1-yl, 4-Ethyl-piperazin-1-yl, 4-Acetyl-piperazin-1-yl, 4-((5-Methyl-2-oxo-1,3-dioxol-4-yl)-methyl-piperazin-1-yl, 4-(3-Carboxy-1-oxo-propyl)-piperazin-1-yl, 3,5-Dimethyl-piperazin-1-yl, 2,4,5-Trimethyl-piperazin-1-yl, 3,4,5-Trimethyl-piperazin-1-yl, 2-Aminomethylmorpholin-4-yl, 2-Dimethylaminomethyl-morpholin-4-yl, 3-Methylaminomethylmorpholin-4-yl, 7-Amino-5-azaspiro(2.4)heptan-5-yl, 8-Amino-6-azaspiro(3.4)octan-6-yl, 6-Amino-3-azabicyclo(3.1.0)hexan-3-yl, 6-Alanylalanylamino-3-azabicyclo(3.1.0)hexan-3-yl, 6-Amino-1-methyl-3-azabicyclo-(3.2.0)heptan-3-yl, 6-Methyl-2,5-diazabicyclo(2.2.1)heptan-2-yl, 2,5-Diazabicyclo(2.2.1)heptan-2-yl, 8-Methyl-3-8-diaza-bicyclo(3.2.1)octan-3-yl, 5-Amino-2-aza-2-spiro[4.4]nonyl, 1-Aminomethyl-8-aza-8-bicyclo-[4.3.0]nonyl, 5-Aminomethyl-7-aza-2-oxo-7-bicyclo[3.3.0]octyl, 1-Aminomethyl-7-aza-3-oxo-7-bicyclo[3.3.0]octyl, 2,7-Diaza-7-bicyclo[3.3.0]octyl, 3,7-Diaza-3-bicyclo[3.3.0]octyl, 2,8-Diaza-8-bicyclo[4.3.0]nonyl, 5,8-Diaza-2-oxo-8-bicyclo[4.3.0]nonyl, 3,8-Diaza-8-bicyclo[4.3.0]nonyl, 2,7-Diaza-7-bicyclo[4.3.0]nonyl, 3,9-Diaza-9-bicyclo[4.3.0]nonyl, 3,9-Diaza-3-bicyclo[4.3.0]nonyl, 7-Amino-3-aza-3-bicyclo[4.1.0]heptyl, 7-Amino-5-azaspiro[2.4]hept-5-yl oder 7-Methylamino-5-azaspiro[2.4]hept-5-yl, 2,7-Diaza-2-bicyclo[3.3.0]octyl, 4-Amino-1,3-dihydro-2H-isoindol-2-yl, 3,4-Dihydro-2(1 H)-isochinolinyl, Hexahydropyrrolo[3,4-b]pyrrol-5(1 H)-yl, Octahydro-6H-pyrrolo[3,4-b]pyridin-6-yl, Hexahydropyrrolo[3,4-b]-1,4-oxazin-6(2H)-yl, 2,3-Dihydro-1-methyl-1H-isoindol-5-yl, Pyridin-4-yl, 2,6-Dimethylpyridin-4-yl, 1 H-Pyrrol-1-yl oder 1H-Imidazol-1-yl.

Beispiele für Verbindungen der Formel (I) sind 1-Cyclopropyl-6-fluor-1,4-dihydro-8-methoxy-7-[3-(methylamino)-1-piperidinyl]-4-oxo-3-chinolincarbonsäure (Balofloxacin), 8-Chlor-1-cyclopropyl-6-fluor-1,4-dihydro-7-[(4aS,7aS)-octahydro-6H-pyrrolo[3,4-b]pyridin-6-yl]-4-oxo-3-chinolincarbonsäure Monohydrochlorid (BAY Y3118), 1-Cyclopropyl-6-fluor-8-difluormethoxy-1,4-dihydro-7-((3S)-methyl-1-piperazinyl)-4-oxo-3-chinolincarbonsäure Hydrochlorid (Caderofloxacin), 1-Cyclopropyl-6-fluor-1,4-dihydro-4-oxo-7-(1-piperazinyl)-3-chinolincarbonsäure (Ciprofloxacin), 7-(3-Amino-1-pyrrolidinyl)-8-chlor-1-cyclopropyl-6-fluor-1,4-dihydro-4-oxo-3-chinolincarbonsäure (Clinafloxacin), (1α, 5α, 6β)-(+)-7-(6-Amino-1-methyl-3-azabicyclo[3.2.0]hept-3-yl)-1-cyclopropyl-6-fluor-1,4-dihydro-4-oxo-1,8-naphthyridin-3-carbonsäure (Ecenofloxacin), 1-Cyclopropyl-7-(4-ethyl-1-piperazinyl)-6-fluor-1,4-dihydro-4-oxo-3-chinolincarbonsäure (Enrofloxacin), 1-Cyclopropyl-6-fluor-1,4-dihydro-8-methoxy-7-(3-methyl-1-piperazinyl)-4-oxo-3-chinolincarbonsäure (Gatifloxacin), 7-[(4Z)-3-(Aminomethyl)-4-(methoxyimino)-1-pyrrolidinyl]-1-cyclopropyl-6-fluor-1,4-dihydro-4-oxo-1,8-naphthyridin-3-carbonsäure (Gemifloxacin), 1-Cyclopropyl-6-fluor-1,4-dihydro-5-methyl-7-(3-methyl-1-piperazinyl)-4-oxo-3-chinolincarbonsäure (Grepafloxacin), 1-Cyclopropyl-6-fluor 1,4-dihydro-8-methoxy-7-[(4aS,7aS)-octahydro-6H-pyrrolo[3,4-b]pyridin-6-yl]-4-oxo-3-chinolincarbonsäure (Moxifloxacin), 5-Amino-7-[(7S)-7-amino-5-azaspiro[2.4]hept-5-yl]-1-cyclopropyl-6-fluor-1,4-dihydro-8-methyl-4-oxo-3-chinolincarbonsäure (Olamufloxacin), oder 7-[(7S)-7-Amino-5-azaspiro[2.4]hept-5-yl]-8-chloro-6-fluor-1-[(1 R,2S)-2-fluorcyclopropyl]-1,4-dihydro-4-oxo-3-chinolincarbonsäure (Sitafloxacin).

Sofern entsprechende physiologisch verträgliche Säureadditionssalze der Verbindungen der allgemeinen Formel (I) zum Einsatz kommen, können diese vorzugsweise aus der Gruppe Hydrochlorid, Hydrobromid, Methansulfonat und Toluolsulfonat ausgewählt werden. Sofern entsprechende physiologisch verträgliche Salze der zugrundeliegenden Carbonsäuren zum Einsatz kommen, können diese bevorzugt aus der Gruppe der Alkalisalze, Erdalkalisalze, Ammoniumsalze, Guanidiniumsalze oder Silbersalze ausgewählt werden. Es können auch Gemische aus wenigstens zwei der vorstehend genannten physiologisch verträglichen Salze zum Einsatz kommen.

Die vorstehend genannten Verbindungen der Formel (I) sind bekannt und können nach üblichen, dem Fachmann bekannten Verfahren hergestellt werden. Ebenso ist bekannt, dass die Verbindungen der Formel (I) antibiotisch wirken und ein antibakterielles Spektrum haben gegen grampositive und gramnegative Keime. Auch ist bekannt, dass die Verbindungen der Formel (I) zur systemischen Behandlung von Erkrankungen eingesetzt werden können, die durch gramnegative oder grampositive oder bakterienähnliche Mikroorganismen hervorgerufen werden können. Es ist auch bekannt, dass Ciprofloxacin in topischer Form in der Augenheilkunde angewendet werden kann.

Die Verbindungen der allgemeinen Formel (I) können zur topischen und/oder lokalen Behandlung von verschiedenen bakteriell verursachten Erkrankungen und zur Prophylaxe bei Mensch und Tier verwendet werden.

Die Verbindungen der Formel (I) können insbesondere in der Zahnmedizin und/oder zur Verbesserung der Wundheilung in der Allgemeinmedizin oder Tiermedizin verwendet werden. Die erfindungsgemäßen Verwendungen der Verbindungen der Formel (I) betreffen bevorzugt d) die topische und/oder lokale Behandlung von postoperativen oder posttraumatischen Wundinfektionen und e) bei der periooperativen Prophylaxe, f) bei infizierten Verbrennungen, g) bei Handinfektionen, h) bei der postoperativen Sepsis, i) bei infizierten Ulzera und Gangränen, j) bei akuten und chronischen bakteriellen Hautinfektionen, k) bei sekundär infizierten Dermatosen, I) bei der Akne und Rosazea oder m) bei Schleimhautulzerationen.

Im folgenden werden bevorzugte Verwendungen gemäß der Erfindung beispielhaft auf dem Gebiet der Wundheilung beschrieben.

### Wundversorgung

Postoperative Wundinfektionen können allgemein bei unzureichender Infektionsprophylaxe nach chirurgischen Eingriffen auftreten. Posttraumatische Wundinfektionen können durch Schnitte, Stiche, Quetschungen, Bisse oder Schüsse hervorgerufen werden. Eine lokale, perioperative Prophylaxe kann bei aseptischen Operationen mit leichtem Infektionsrisiko erfolgen. Zusätzlich zur systemischen perioperativen Prophylaxe kann auch eine lokale perioperative Prophylaxe angewandt werden, zum Beispiel bei Infektionen mit erhöhtem Infektionsrisiko wie Implantationen, bei Herzoperationen, bei Transplantationen, bei neurochirurgischen Operationen, bei Operationen in stark kontaminiertem Gebiet wie Mundhöhle, Ösophagus, Rektum oder Kolon, bei Hysterektomie, bei Gallenwegsoperationen, bei Operationen von Patienten mit Abwehrschwäche oder bei Amputationen. Die lokale Behandlung von Verbrennungen des ersten, zweiten oder dritten Grades kann prophylaktisch oder nach Infektion erfolgen. Besonders bei schweren Verbrennungen ist die antibakterielle Lokalbehandlung von großem Nutzen. Handinfektionen sind beispielsweise Panaritium cutaneum, Panaritium subcutaneum, Panaritium ossale, Panaritium articulare oder Tendovaginitis purulenta. Bei der postoperativen Sepsis können die infizierten Wunden durch topische Gabe der antibakteriellen Mittel weitgehend oder vollständig keimfrei gemacht werden. Gangräne können zusätzlich zur Therapie mit parenteralen Antibiotika durch lokale Behandlung mit Antibiotika behandelt werden. Akute bakterielle Hautinfektionen sind beispielsweise Pyodermien, Erysipel, Furunkel, Karbunkel, Phlegmonen, Abszesse, Ulcus cruris, der diabetische Fuß, infizierte Dekubitalulzera, hämorrhagische Blasen, Erysipeloide, oder Erythrasma. Beispiele chronischer bakterieller Hautinfektionen sind Lupus vulgaris, Schwimmbad-Granulome, Buruli-Ulkus oder Aktinomykose. Bakterielle Sekundärinfektionen treten beispielsweise bei Virusinfektionen wie Herpes simplex, Herpes zoster oder Varizellen auf. Bakterielle Sekundärinfektionen von Dermatosen treten beispielsweise bei Ekzemen, Neurodermitis im Exsudationsstadium, blasenbildenden Dermatosen oder Kontaktdermatitis auf. Leichtere und mittlere Formen der Akne und Rosazea können auch lokal behandlet werden. In allen genannten Fällen kann die lokale Gabe der Verbindungen der Formel (I), entweder allein oder aber additiv zu einer systemischen Applikation erfolgen.

Die lokale Gabe der Verbindungen der Formel (I) hat sich als vorteilhaft erwiesen, da wegen der breiten Wirksamkeit dieser Verbindungen vorteilhaft auch Mischinfektionen behandelt werden können. Bisherige topisch applizierte Antibiotika haben nur ein eingeschränktes Wirkspektrum und sind damit weniger wirksam. Weitere Vorteile der Verbindungen der Formel (I) sind der sehr schnelle Eintritt der antibakteriellen Wirkung. Dies ermöglicht einen therapeutischen Erfolg schon während des Aufenthaltes des Patienten in der Praxis. Diese Verbindungen der Formel (I) haben eine ausgeprägte bakterizide Wirkung und sind damit den heutigen, topisch applizierten Antibiotika überlegen, die oft nur eine bakteriostatische Wirkung haben und demnach wesentlich häufiger und länger angewendet werden müssen. Weitere Vorteile der Verbindungen der Formel (I) gegenüber heutigen Lokalantibiotika ist ihre gute Gewebegängigkeit. Ihre Penetration durch die intakte Haut ermöglicht auch die erfolgreiche örtliche Lokalbehandlung von tiefer liegenden Hautinfektionen. Ebenfalls als Vorteil der Verbindungen der Formel (I) ist anzusehen. dass sie ein wesentlich geringeres Potential zur bakteriellen Resistenzbildung aufweisen als herkömmliche Lokalantibiotika. Dadurch lassen sie sich wesentlich sicherer anwenden. Ein weiterer Vorteil der Verbindungen der Formel (I) ist eine allgemein zu beobachtende beschleunigende Wirkung auf die Wundheilung. Zusätzliche Vorteile der lokalen Verwendung von Verbindungen der Formel (I) sind die Verhinderung von Komplikationen wie Lymphangitis, Sepsis oder chronische Lokalinfektionen.

Die besondere Wirksamkeit der Verbindungen der Formel (I) bei topischer Applikation hat sich überraschenderweise auch bei der Therapie des diabetischen Fuß-Syndroms gezeigt. Nach dem heutigen Stand der Medizin gilt, dass jede Läsion mit entzündlichem- Lokalbefund mit und ohne systemische Infektionszeichen einer sofortigen und breitabdeckenden Antibiose bedarf. Dabei ist zu berücksichtigen, daß bei dem entzündlichen Geschehen regelhaft eine Mischinfektion von grampositiven und gramnegativen Keimen sowie von Anaerobiern und Aerobiem vorliegt. Initial hat sich die systemische Gabe von Amoxicillin, Clavulansäure oder Clindamycin jeweils in Kombination mit einem Gyrasehemmer bewährt. Entsprechend des Ergebnisses des vor Antibiotikagabe abgenommenen Wundabstriches kann dann die Antibiose gezielt durchgeführt werden. Kontrovers wird jedoch die notwendige Dauer der Antibiotikatherapie, insbesondere bei osteomyelitischen Defekten, diskutiert. Eine monatelange systemisch hochdosierte Antibiotikagabe erscheint sinnlos, wenn radiologisch eine nachweisbare Abheilungstendenz der Osteolysen nicht zu erkennen ist; hier wird eine chirurgische Intervention unumgänglich sein.

Bei der heute üblichen Therapie des diabetischen Fußes gilt, dass eine essentielle Voraussetzung für eine komplikationslose Abheilung eine infektionsfreie Wunde ist. Daher ist beim Vorliegen einer infizierten Wunde die schnelle und zuverlässige Infektionsbehandlung oberstes Gebot. Eine lokale oder systemische Antibiotikatherapie birgt das Risiko der Allergie und der Resistenzentwicklung. Besonders bewährt haben sich Verbände, bestehend aus Aktivkohle und elementarem Silber. Das nichttoxische elementare Silber erlaubt eine sehr starke lokale Infektionsbekämpfung. Die Aktivkohle bindet Mikroorganismen und Zelldetritus und erlaubt das Entfernen der ungewünschten Partikel beim Verbandwechsel. Resistenzentwicklungen, lokale Reizungen oder Allergien sind dabei ausgeschlossen, gleichzeitig wird das notwendige feuchte Milieu gewährleistet. Als Desinfektionsmittel haben Farbstoffe bei der modernen Wundbehandlung, mit Ausnahme von PVP-Jod-Komplexen, keine Bedeutung mehr. Kaliumpermanganat ist problematisch in der Dosierung und kann zu schweren Hautverätzungen führen. Ethacridinlaktat weist eine hohe Allergierate und nur eine beschränkte antimikrobielle Wirksamkeit auf. Das quecksilberhaltige Merbromin ist hochtoxisch, beeinträchtigt die Granulation und ist problematisch bei der Entsorgung. Andere Farbstoffe, wie z.B. Brillantgrün, Methylviolett und Fuchsin, sind wegen ihrer geringen Wirksamkeit, vor allem aber wegen des epithelschädigenden Effektes obsolet.

Demnach besteht nach dem heutigen Stand der Technik auch bei der Behandlung des diabetischen Fußes ein Bedürfnis nach topisch und/oder lokal anwendbaren antibiotisch wirksamen Mitteln, die eine hohe Aktivität gegen die in Wunden auftretenden Keime aufweisen, die schnell wirksam sind, die lokal gut verträglich sind, die eine geringe Neigung zur Erzeugung antibiotischer Resistenz hervorrufen, die bei topischer und/oder lokaler Anwendung einfach zu verabreichen sind, die bei topischer/lokaler Gabe eine minimale systemische Belastung des Organismus bewirken, die eine gute Gewebegängigkeit aufweisen, die auch zur Behandlung von tiefen Infektionen geeignet sind und die darüber hinaus den Prozess der Wundheilung beschleunigen.

Es wurde nun gefunden, dass auch bei der topischen Applikation der Verbindungen der Formel (I) ein Erfolg bei der Therapie des diabetischen Fuß-Syndroms erzielt werden konnte. Dies zeigte sich besonders darin, dass bei Patienten mit schweren Mikroangiopathien der Füße mit diabetischen Fußsyndromen die systemischen Behandlungsversuche mit Avalox 400 (Moxifloxacin) und/oder Clont 400 (2-Methyl-5-nitro-1H-imidazole-1-ethanol) ohne nennenswerte Befundänderungen verliefen, da diese Wirkstoffe wegen ausgeprägter Mikroangiopathien nach systemischer Gabe nur sehr geringe Gewebekonzentrationen erreichen konnten. Überraschenderweise führte die topische Applikation der Verbindungen der Formel (I) jedoch zu signifikanten Verbesserungen des Krankheitsgeschehens. Es konnten Ulcera unterschiedlicher Schweregrade (Ulcera D I bis D V) behandelt werden. Schmierige Ulcera wurden sauber, verkrusteten, ihre Größe verringerte sich und sie heilten sogar vollkommen ab. Diese Behandlungserfolge wurde erzielt nach mehrmaliger topischer Applikation der Verbindungen der Formel (I) in wöchentlichem Abstand. Dabei konnten die Verbindungen der Formel (I) direkt in gelöster Form oder beispielsweise als Gel oder mittels getränkter Kompressen oder Verbände auf die Wunde aufgetragen werden. Diese Verbindungen der Formel (I) können in Konzentrationen von 0,005 mg/mL bis 200 mg/mL auf die Wunde aufgetragen werden, bevorzugte Konzentrationsbereiche für Lösungen oder Gele sind solche von 0,1 bis 150 mg/mL. Bei getränkten Kompressen oder Verbänden können gelförmige Darreichungsformen in den Konzentration von 25-150 mg/mL verwendet werden.

Es wurde ferner gefunden, dass die lokale und/oder topische Anwendungen der Verbindungen der Formel (I) auch in Tiermedizin von Nutzen ist.

Die Verbindungen der Formel (I) können auch in Kombinationen mit anderen Antiinfektiva wie antibakteriellen, antifungalen oder antiviralen Wirkstoffen eingesetzt werden.

Die Verbindungen der Formel (I) können in Konzentrationen von 0,005 mg/mL bis 200 mg/mL eingesetzt werden. Bevorzugte Konzentrationen sind solche im Bereich von 0,5 mg/mL bis 200 mg/mL. Besonders bevorzugte Konzentrationen sind solche im Bereich von 10 bis 150 mg/mL.

Die Verbindungen der Formel (I) können als Lösungen, Gele, Suspensionen, Emulsionen, Liposomen oder in Mizellen eingesetzt werden. Lösungen sind beispielsweise Lösungen in Wasser oder wässrige Lösungen in Gegenwart von Löslichkeitsverbesserem. Löslichkeitsverbesserer sind beispielsweise Salze, Polyole, Zuckeralkohole, Polyglycole oder Kosolventien wie Glycerin, Ethylenglycol, Propylenglycol, Furfural, N,N-Dimethylfomamid, Methanol, Ethanol, i-Propanol, n-Propanol oder Aceton. Wässrige Gele werden durch Zusatz von Gelbildnern wie beispielsweise Pektinen, Ethylenglycolmonomethacrylatgel, Alginaten, Hydroxyethylcellulose, Carboxymethylhydroxyethylcellulose, Polyglycerylmethacrylate oder Polysacchariden hergestellt. Geeignete Zusätze sind auch Verdicker wie Cellulose, Alkylcellulose, Hydroxyethylcellulose, Agar-Agar, Carboxymethyl-Guar, Celluloseether oder hydrotrope Lösungsvermittler wie Ethylendiamin, Harnstoff oder Cyclodextrine. Die galenischen Formen können auch Solubilisatoren wie Tenside oder Konservierungsmittel enthalten. Bestandteile von Suspensionen können beispielsweise Traganth, Cellulose, Netzmittel, Glycole, Polyole, Schleimstoffe oder Celluloseether sein. Bestandteile von Emulsionen können Emulgatoren wie Polysorbate, Tenside, Lecithine, Schleimstoffe, Gelatine oder Carboxymethylcellulose sein. Geeignete Formen der Verabreichung können auch Inserte zum Einlegen in die Zahntaschen sein, die aus einem inerten Trägermaterial bestehen und die mit dem Wirkstoff und gegebenenfalls weiteren Hilfsstoffen imprägniert sind und den Wirkstoff unter Herauslösen allmählich freigeben. Formen der Verabreichung für Wurzelfüllungen können beispielsweise Tampons, Wattebausche oder Schaumstoffpellets sein. Die Applikation bei Weichteilinfektionen kann mit Streifen oder Fadeneinlagen erfolgen. Es können auch Arzneistoffe oder Hilfsstoffe zur osmotischen Angleichung verwendet werden. Weitere Hilfsstoffe zur Formulierung der Verbindungen der Formel (I) können Antioxidantien, Chelatbildner, Desinfektionsmittel, Dispergiermittel, Emulsionsstabilisatoren, Hydrokolloide, Konservierungsmittel, Lösungsvermittler, Netzmittel, quartäre Ammoniumverbindungen, Stabilisatoren, Suspendiermittel oder Verdickungsmittel sein. Die vorstehend genannten Bestandteile können auch in Kombinationen untereinander verwendet werden.

Geeignete stabile gelartige Formulierungen sind solche, die neben den Verbindungen der Formel (I) aus Polyethern und modifizierten Cellulosen und Wasser aufgebaut sind. Bevorzugte Formulierungen für Gele sind solche, die die Verbindungen der Formel (I) in Gemischen aus Propylenglycol, Tween-20-Lösung und Muc. Hydroxyethylcell. enthalten. Bevorzugte Zusammensetzungen bestehen aus Verbindungen der Formel (I) in Mengen von 0,001 bis 100 mg/mL, Polypropylenglycol in Mengen von 5 bis 250 mg/mL, Tween-20-Lösung 1 % in Mengen von 5 bis 200 mg/mL und Muc. Hydroxyethylcell. ad 1 g/mL enthalten. Besonders bevorzugte gelartige Formulierungen bestehen aus den Verbindungen der Formel (I) mit 1 bis 100 mg/mL, Propylenglycol mit 50 bis 200 mg/mL, Tween-20-Lösung 1 % mit 3 bis 150 mg/mL und Muc. Hydroxycell ad 1 g/mL.

Die folgenden Beispiele dienen der Erläuterung der vorliegenden Erfindung, schränken aber den allgemeinen Erfindungsgedanken nicht ein.

### BEISPIELE

### Beispiele verschiedener Formulierungen

### Beispiel 1:

### Moxifloxacin-HCl-Lösung

| | |
|---|---|
| Moxifloxacin-Hydrochlorid | 500 mg |
| aqua pro injectione | ad 100 ml |

### Beispiel 2:

### hochviskose Formulierung: Moxifloxacin-HCl-Gel

| | |
|---|---|
| Moxifloxacin-Hydrochlorid | 1,0 g |
| Hydroxyethylcellulose | 0,5 g |
| Propylenglycol | 1,5 g |
| dest. Wasser zu | 10,0 g |

### Beispiel 3:

### niedrigviskose Formulierung mit Stabilisator Moxifloxacin-HCl-Gel

| | |
|---|---|
| Moxifloxacin-Hydrochlorid | 1,0 g |
| Hydroxyethylcellulose | 0,25 g |
| Propylenglycol | 1,5 g |
| Tween-20-Lösung 1 %-ig | 1,0 g |
| dest. Wasser zu | 10,0 g |

### Beispiel 4:

### Moxifloxacin-HCl-Gel

| | |
|---|---|
| Moxifloxacin-Hydrochlorid | 0,1 g |
| Hydroxyethylcellulose | 0,25 g |
| Propylenglycol | 1,5 g |
| Tween-20-Lösung 1 %-ig | 1,0 g |
| dest. Wasser zu | 10,0 g |

### Referenzbeispiele A1.1-A3.23. Endodontische Erkrankungen

### A.1. Topische Behandlung von Pulpitiden auf Grund von kariösen Erkrankungen

**Patient A 1.1** (männlich, 43 Jahre). Klinische Diagnose: Zahn 24, kariöser mesialer Defekt (Schmelz/Dentin), Zahn vital, verstärkt Schmerzen auf kalt. Behandlungsverlauf: Entfernung der Karies. Restdentindecke geringfügig erweicht. Pelleteinlage getränkt mit Moxifloxacin-Hydrochlorid-Gel (25 mg/mL), provisorischer Kavitätenverschluss. Kontrolluntersuchung nach 4 Tagen: Patient war beschwerdefrei. Unterfüllung mit Dropsin, Abschlussfüllung mit Composite. Kontrolluntersuchung nach 3 Wochen: Zahn vital, Patient beschwerdefrei.

**Patient A 1.2** (männlich, 33 Jahre). Klinische Diagnose: Zähne 11, 13, größere distale und mesiale kariöse Defekte (Schmelz/Dentin), alle Zähne vital, Zahn 11 mit Schmerzen auf kalt, Zahn 13 mit leichten Beschwerden auf warm. Behandlungsverlauf: Entfernung der Karies. Restdentindecke hart. Pelleteinlagen mit Moxifloxacin-Hydrochlorid-Gel (50 mg/mL), provisorischer Kavitätenverschluß. Kontrolluntersuchung nach 1 Woche: Zähne 11 und 13 beschwerdefrei. Unterfüllung mit Dropsin, Abschlußfüllung mit Composite Kontrolluntersuchung nach 14 Tagen: Zähne vital, Patient beschwerdefrei.

**Patient A 1.3** (weiblich, 18 Jahre). Klinische Diagnose: Zahn 24, großer distaler kariöser Defekt (Schmelz/Dentin), Zahn vital, Schmerzen auf kalt, süß und sauer. Behandlungsverlauf: Entfernung der Karies. Restdentindecke hart. Pelleteinlage mit Moxifloxacin-Hydrochlorid-Gel (25mg/mL), provisorischer Kavitätenverschluß. Kontrolluntersuchung nach einer Woche: noch unklare, geringe Beschwerden, Wiederholung der Einlage. Kontrolluntersuchung nach 3 Tagen: Zahn vital, Patient beschwerdefrei. Unterfüllung mit Dropsin, Abschlußfüllung Glasionomer.

**Patient A 1.4** (männlich, 23 Jahre). Klinische Diagnose: Zahn 11, großer kariöser mesialer Defekt, Zahn vital, Schmerzen auf warm und kalt, Nachtschmerz. Behandlungsverlauf: Entfernung der Karies. Restdentindecke leicht erweicht. Pelleteinlagen mit Moxifloxacin-Hydrochlorid-Gel (50mg/mL), provisorischer Kavitätenverschluß. Kontrolluntersuchung nach 7 Tagen: Patient beschwerdefrei, Zahn vital. Unterfüllung Zinkphosphatzement, Abschlußfüllung Glasionomer.

**Patient A 1.5** (männlich, 43 Jahre). Klinische Diagnose: Zahn 13, großer distaler kariöser Defekt, Zahn schwach vital, Schmerzen auf kalt, Nachtschmerz. Behandlungsverlauf: Entfernung der Karies. Restdentindecke hart. Pelleteinlage mit Moxifloxacin-Hydrochlorid-Gel (50 mg/mL), provisorischer Kavitätenverschluß. Kontrolluntersuchung nach 4 Tagen: Patient beschwerdefrei, Zahn vital. Unterfüllung Dropsin, Abschlußfüllung Composite.

**Patient A 1.6** (weiblich, 42 Jahre). Klinische Diagnose: Zahn 36, großer mesialer kariöser Defekt, Zahn mit verringerter Vitalität, Schmerzen auf kalt und warm, Nachtschmerz. Behandlungsverlauf: Entfernung der Karies, Pelleteinlage mit Moxifloxacin-Hydrochlorid-Gel (50 mg/mL), provisorischer Kavitätenverschluß. Kontrolluntersuchung nach 6 Tagen: noch geringe Schmerzen auf warm und kalt. Wiederholung der Einlage. Kontrolluntersuchung nach 7 Tagen: Patient beschwerdefrei, Zahn vital. Unterfüllung Zinkphosphatzement, Abschlußfüllung Composite.

**Patient A 1.7** (weiblich, 18 Jahre). Klinische Diagnose: Zahn 44, großer mesialer kariöser Defekt, Zahn vermindert vital, Schmerzen auf kalt, Nachtschmerz. Behandlungsverlauf: Entfernung der Karies. Restdentindecke leicht erweicht. Pelleteinlagen mit Moxifloxacin-Hydrochlorid-Gel (50 mg/mL), provisorischer Kavitätenverschluß. Kontrolluntersuchung nach 4 Tagen: Patient beschwerdefrei, Unterfüllung Zinkphosphatzement, Abschlußfüllung Composite.

**Patient A 1.8** (männlich, 38 Jahre). Klinische Diagnose: Zahn 36, großer distaler kariöser Defekt, Zahn vermindert vital, Schmerzen auf kalt, warm, süß, sauer, leicht perkussionsempfindlich, Nachtschmerz. Behandlungverlauf: Entfernung der Karies. Restdentindecke leicht erweicht. Pelleteinlage mit Moxifloxacin-Hydrochlorid-Gel (50 mg/mL), provisorischer Kavitätenverschluß.

Kontrolluntersuchung nach 3 Wochen: Patient beschwerdefrei, Zahn vital, Unterfüllung Zinkphosphat, Abschlußfüllung Composite.

**Patient A 1.9** (weiblich, 50 Jahre). Klinische Diagnose: Zahn 27, großer distaler kariöser Defekt, Zahn vital, Beschwerden auf kalt, leichte Beschwerden auf warm, leichter Nachtschmerz. Behandlungsverlauf: Entfernung der Karies. Restdentindecke erweicht. Pelleteinlage mit Moxifloxacin-Hydrochlorid-Gel (25 mg/mL), provisorischer Verschluß. Kontolluntersuchung nach 7 Tagen: Patientin beschwerdefrei, Zahn vital.

**Patient A 1.10** (männlich, 17 Jahre). Klinische Diagnose: Zähne 16, 17, 27, 36, 37, 46 okklusal kariös, Zähne vital, Schmerzen auf kalt, süß und sauer, manchmal auf warm, nicht lokalisierbar. Behandlungsverlauf: Entfernung der Karies. Restdentindecken hart. Pelleteinlagen mit Moxifloxacin-Hydrochlorid-Gel (50mg/mL), provisorischer Kavitätenverschluß. Kontrolluntersuchungen nach 14 Tagen: Patient beschwerdefrei, Zähne vital. Unterfüllung Dropsin, Abschlußfüllung Composite.

**Patient A 1.11** (männlich, 33 Jahre). Klinische Diagnose: Zahn 22, kariöser distaler Defekt (Schmelz/Dentin), Zahn vital, leichte Schmerzen auf warm. Behandlungsverlauf: Entfernung der Karies, Restdentindecke geringfügig erweicht. Pelleteinlage getränkt mit Grepafloxacin-Gel (25 mg/mL), provisorischer Kavitätenverschluß. Kontrolluntersuchung nach 5 Tagen: Patient war beschwerdefrei. Unterfüllung mit Zink-Phosphatzement, Abschlußfüllung mit Composite. Kontrolluntersuchung nach 4 Wochen: Zahn vital, Patient beschwerdefrei.

**Patient A 1.12** (männlich, 38 Jahre). Klinische Diagnose: Zähne 22, 23 mit größeren distalen kariösen Defekten (Schmelz/Dentin), Zähne vital, Schmerzen auf kalt. Behandlungsverlauf: Entfernung der Karies, Restdentindecke hart. Pelleteinlagen mit Grepafloxacin-Gel (50 mg/mL), provisorischer Kavitätenverschluß. Kontrolluntersuchung nach 1 Woche: Zähne beschwerdefrei. Unterfüllung mit Dropsin, Abschlußfüllung mit Composite. Kontrolluntersuchung nach 14 Tagen: Zähne vital, Patient beschwerdefrei.

**Patient A 1.13** (weiblich, 48 Jahre). Klinische Diagnose: Zahn 14, großer mesialer kariöser Defekt (Schmelz/Dentin), Zahn vital, Schmerzen auf kalt und sauer. Behandlungsverlauf: Entfernung der Karies, Restdentindecke hart. Pelleteinlage mit Gemifloxacin-Mesylat-Gel (25 mg/mL), provisorischer Kavitätenverschluß. Kontrolluntersuchung nach 3 Wochen: noch geringe Beschwerden. Wiederholung der Einlage. Kontrolluntersuchung nach 4 Tagen: Zahn vital, Patient beschwerdefrei. Unterfüllung mit Dropsin, Abschlussfüllung mit Glasionomer.

**Patient A 1.14** (männlich, 53 Jahre). Klinische Diagnose: Zahn 13, großer kariöser mesialer Defekt, Zahn vital, Schmerzen auf warm, Nachtschmerz. Behandlungsverlauf: Entfernung der Karies, Restdentindecke leicht erweicht. Pelleteinlagen mit Levofloxacin-Gel (50 mg/mL), provisorischer Kavitätenverschluß. Kontrolluntersuchung nach 14 Tagen: Patient beschwerdefrei, Zahn vital. Unterfüllung mit Dropsin, Abschlussfüllung mit Composite

**Patient A 1.15** (männlich, 22 Jahre). Klinische Diagnose: Zahn 44, großer mesialer kariöser Defekt, Zahn vital, Schmerzen auf kalt, Nachtschmerz. Behandlungsverlauf: Entfernung der Karies, Restdentindecke lederartig. Pelleteinlage mit Trovafloxacin-Mesylat-Gel (50 mg/mL), provisorischer Kavitätenverschluß. Kontrolluntersuchung nach 9 Tagen: Patient beschwerdefrei, Zahn vital. Unterfüllung mit Dropsin, Abschlussfüllung mit Composite.

**Patient A 1.16** (weiblich, 15 Jahre). Klinische Diagnose: Zahn 46, großer distaler kariöser Defekt, Zahn mit verringerter Vitalität, Schmerzen auf kalt und warm. Behandlungsverlauf: Entfernung der Karies, Pelleteinlage mit Sparfloxacin-Gel (25 mg/mL), provisorischer Kavitätenverschluß. Kontrolluntersuchung nach 4 Tagen: noch geringe Schmerzen auf warm. Wiederholung der Einlage. Kontrolluntersuchung nach 14 Tagen: Patientin beschwerdefrei, Zahn vital. Unterfüllung mit Dropsin, Abschlussfüllung mit Composite.

### A.2. Prophylaxe von Dentinwunden

**Patient A 2.1** (männlich, 30 Jahre). Klinische Diagnose: Zähne 33 und 36, kariöse Defekte an beiden Zähnen. Zähne vital. Behandlungsverlauf: Präparation der Zähne 33 und 36. Anfertigung einer provisorischen Brücke. Auftrag von Moxifloxacin-Hydrochlorid-Lösung (25 mg/mL) auf Kavität, Trocknung im leichten Luftstrom. Befestigung der Brücke mit provisorischem Zement. Nach 8 Tagen definitives Einsetzen der Brücke mit Zink-Phosphatzement. Kontrolluntersuchung nach 3 Wochen: Patient beschwerdefrei.

**Patient A 2.2** (männlich, 48 Jahre). Klinische Diagnose: Zähne 22, 23, 24 mit kleinen distalen kariösen Defekten (Schmelz/Dentin), alle Zähne vital, Schmerzen auf kalt. Behandlungsverlauf: Entfernung der Karies, Vorstrich mit Moxifloxacin-Hydrochlorid-Lösung (50 mg/mL), provisorischer Kavitätenverschluß. Kontrolluntersuchung nach 2 Wochen: Zähne beschwerdefrei. Unterfüllung mit Dropsin, Abschlußfüllung mit Composite. Kontrolluntersuchung nach 4 Wochen: Zähne vital, Patient beschwerdefrei.

**Patient A 2.3** (weiblich, 28 Jahre). Klinische Diagnose: Zähne 14, 15, 16, 17 mit cervikalen Erosionen (Schmelz/Dentin), Schmerzen auf kalt und süß. Behandlungsverlauf: Präparation, Vorstrich mit Moxifloxacin-Hydrochlorid-Lösung (50 mg/mL), sanfte Trocknung im Luftstrom. Adhäsive Restauration mit Composite. Kontrolluntersuchung nach 3 Wochen: Patientin beschwerdefrei.

**Patient A 2.4** (männlich, 33 Jahre). Klinische Diagnose: Zähne 11, 12, 21, 22 mit kariösen Defekten im ersten Dentindrittel, Zähne vital. Behandlungsverlauf: Präparation für Keramikkronen. Vorstrich mit Moxifloxacin-Hydrochlorid-Lösung (25 mg/mL), Trocknung im sanften Luftstrom, Einsetzen von provisorischen Kronen. Nach 14 Tagen Eingliedern der definitiven Kronen mit Zinkphosphatzement. Kontrolluntersuchung nach 3 Wochen: Patient beschwerdefrei.

**Patient A 2.5** (männlich, 62 Jahre). Klinische Diagnose: Zahn 44, mesialer kariöser Defekt im ersten Dentindrittel, Schmerzen auf kalt. Behandlungsverlauf: Entfernung der Karies, Vorstrich mit Moxifloxacin-Hydrochlorid-Lösung (25 mg/mL), provisorischer Kavitätenverschluß. Kontrolluntersuchung nach 9 Tagen: Patient beschwerdefrei. Unterfüllung Dropsin, Abschlußfüllung Composite.

**Patient A 2.6** (weiblich, 18 Jahre). Klinische Diagnose: Zahn 43, distaler kariöser Defekt im ersten Dentindrittel. Behandlungsverlauf: Entfernung der Karies, Vorstrich mit Moxifloxacin-Hydrochlorid-Lösung (25 mg/mL), Unterfüllung Dropsin, Abschlußfüllung Composite. Patient zur Kontrolluntersuchung nicht erschienen.

**Patient A 2.7** (weiblich, 28 Jahre). Klinische Diagnose: Zähne 15, 14, 13, 12, 11, 21, 22, 23, 24, 25 mit cervikalen Erosionen, starke Hypersensibilität. Behandlungsverlauf: Reinigung der Erosionsbereiche, Vorstrich mit Sparfloxacin-Lösung (50 mg/mL), sanfte Trocknung im Luftstrom. Adhäsive Restauration mit Composite.

**Patient A 2.8** (weiblich, 33 Jahre). Klinische Diagnose: Zähne 13, 14, 15 mit kariösen Defekten mesial und distal im ersten Dentindrittel, Schmerzen auf kalt. Behandlungsverlauf: Präparation der Kavitäten, Entfernung der Karies, Vorstrich mit Trovafloxacin-Mesylat-Lösung (25 mg/mL), Trocknung im sanften Luftstrom. Adhäsive Restauration mit Glasionomerzement.

### A.3. Topische Behandlung des infizierten Wurzelkanals und des periapikalen Bereichs

**Patient A 3.1** (weiblich, 40 Jahre). Klinische Diagnose: Zahn 45, apikale Parodontitis basierend auf Pulpitis purulenta, leichte Aufbißbeschwerden. Röntgenologisch leicht erweiterter Parodontalspalt, nicht gangränös. Behandlungsverlauf: Kanalaufbereitung bis zum Apex. Kanaleinlage mit Moxifloxacin-Hydrochlorid-Lösung (100 mg/mL) mittels Spritze, anschließend Nachfüllen von Moxifloxacin-Hydrochlorid-Gel (100 mg/mL), provisorischer Verschluß. Kontrolluntersuchung nach 3 Tagen: Patientin beschwerdefrei, Wurzelfüllung mit Endomethasone. Kontrolluntersuchung nach 14 Tagen: Patientin weiterhin beschwerdefrei, Abschlußfüllung Composite.

**Patient A 3.2** (männlich, 70 Jahre). Klinische Diagnose: Zahn 23, gangränös, Aufbißbeschwerden. Röntgenologisch kein Befund. Behandlungsverlauf: Kanalaufbereitung bis zum Apex, Kanaleinlage mit Moxifloxacin-Hydrochlorid-Lösung (100 mg/mL) mittels Spritze, anschließend Nachfüllen von Moxifloxacin-Hydrochlorid-Gel (100 mg/mL), provisorischer Verschluß. Kontrolluntersuchung nach 14 Tagen: Patient beschwerdefrei, Kanal geruchlos. Wurzelfüllung mit Endomethasone.

**Patient A 3.3** (weiblich, 22 Jahre). Klinische Diagnose: Zahn 11, apikale Parodontitis basierend auf Gangrän, Aufbißbeschwerden. Röntgenologisch erweiterter Parodontalspalt. Behandlungsverlauf: Kanalaufbereitung bis zum Apex, Kanaleinlage mit Moxifloxacin-Hydrochlorid-Lösung (50 mg/mL) mittels Spritze, anschließend Nachfüllen von Moxifloxacin-Hydrochlorid-Gel (50 mg/mL) und intraligamentale Injektion von Moxifloxacin-Hydrochlorid-Lösung (50 mg/mL). Provisorischer Verschluß. Kontrolluntersuchung nach 3 Tagen: Patientin beschwerdefrei, Kanal geruchlos.

**Patient A 3.4** (männlich, 40 Jahre). Klinische Diagnose: Zahn 11, apikale Parodontotis basierend Pulpitis purulenta, nicht gangränös, Aufbißbeschwerden. Röntgenologisch erweiterter Parodontalspalt. Behandlungsverlauf: Kanalaufbereitung bis zum Apex, Wurzelkanaleinlage mit Moxifloxacin-Hydrochlorid-Gel (50 mg/mL), provisorischer Verschluß. Kontrolluntersuchung nach 3 Tagen: Patient beschwerdefrei. Wurzelfüllung mit Endomethasone, provisorischer Verschluß. Kontrolluntersuchung nach 3 Wochen: Patient weiterhin beschwerdefrei. Abschlußfüllung Compsite.

**Patient A 3.5** (weiblich, 43 Jahre). Klinische Diagnose: Zahn 35, apikale Parodontitis basierend auf Gangrän, leichte Aufbißbeschwerden. Röntgenologisch schwache Aufhellung sichtbar. Behandlungsverlauf: Kanalaufbereitung bis zum Apex nicht möglich. Wurzelkanaleinlage mit Moxifloxacin-Hydrochlorid-Lösung (50 mg/mL) mittels Spritze, anschließend Nachfüllen von Moxifloxacin-Hydrochlorid-Gel (50 mg/mL) und intraligamentale Injektion von Moxifloxacin-Hydrochlorid-Lösung (50 mg/mL). Kontrolluntersuchung nach 7 Tagen: kein gangränöser Geruch, keine Aufbißbeschwerden. Wurzelfüllung mit N2 medical. Kontrolluntersuchung nach 7 Tagen: Patientin beschwerdefrei. Abschlußfüllung mit Composite

**Patient A 3.6** (männlich, 29 Jahre). Klinische Diagnose: Zahn 36, apikale Parodontitis basierend auf Gangrän, Aufbißbeschwerden. Röntgenologisch kein Befund. Behandlungsverlauf: Kanalaufbereitung bis zum Apex, Wurzelkanaleinlage mit Moxifloxacin-Hydrochlorid-Gel (50 mg/mL) und intraligamentale Injektion von Moxifloxacin-Hydrochlorid-Lösung (50 mg/mL). Kontrolluntersuchung nach 8 Tagen: kein gangränöser Geruch, Patient beschwerdefrei. Wurzelfüllung mit N2 medical. Kontrolluntersuchung nach 3 Wochen: Patient beschwerdefrei. Abschlußfüllung mit Composite.

**Patient A 3.7** (weiblich, 50 Jahre). Klinische Diagnose: Zahn 14, apikale Parodontitis basierend auf Gangrän. Geringe Aufbißbeschwerden. Röntgenologisch schwache Aufhellung. Behandlungsverlauf: Kanalaufbereitung bis zum Apex, Wurzelkanaleinlage mit Moxifloxacin-Hydrochforid-Gel (100 mg/mL) und intraligamentale Injektion von Moxifloxacin-Hydrochlorid-Lösung (50 mg/mL), provisorischer Verschluß. Kontrolluntersuchung nach 7 Tagen: Patientin beschwerdefrei. Wurzelfüllung mit Endomethasone. Kontrolluntersuchung nach 3 Wochen: Patientin weiterhin beschwerdefrei. Abschlußfüllung mit Composite.

**Patient A 3.8** (weiblich, 42 Jahre). Klinische Diagnose: Zahn 34, apikale Parodontitis basierend auf Gangrän, Aufbißbeschwerden. Röntgenologisch schwache Aufhellung sichtbar. Behandlungsverlauf: Kanalaufbereitung bis zum Apex nicht möglich. Wurzelkanaleinlage mit Moxifloxacin-Hydrochlorid-Gel (50 mg/mL) und intraligamentale Injektion von Moxifloxacin-Hydrochlorid-Lösung (50 mg/mL), provisorischer Verschluß. Kontrolluntersuchung nach 13 Wochen: Patientin beschwerdefrei, Kanal geruchsfrei. Wurzelfüllung mit Endomethasone, Abschlußfüllung mit Glasionomer.

**Patient A 3.9** (weiblich, 17 Jahre). Klinische Diagnose: Zahn 46, apikale Parodontitis basierend auf Pulpitis purulenta, leichte Aufbißbeschwerden, Zahn nicht gangränös. Röntgenologisch leicht erweiterter Parodontalspalt an mesialer Wurzel. Behandlungsverlauf: Kanäle aufbereitet bis zum Apex, Wurzelkanaleinlage mit Moxifloxacin-Hydrochlorid-Gel (100 mg/mL) und intraligamentale Injektion von Moxifloxacin-Hydrochlorid-Lösung (50 mg/mL). Kontrolluntersuchung nach 7 Tagen: Patientin beschwerdefrei. Wurzeifüllung mit Endomethasone, Abschlußfüllung. Patientin zur weiteren Kontrolluntersuchung nicht erschienen.

**Patient A 3.10** (weiblich, 24 Jahre). Klinische Diagnose: Zahn 14, Fistel im Wurzelspitzenbereich, Zahn gangränös. Leichte Aufbißbeschwerden. Behandlungsverlauf: Kanalaufbereitung bis zum Apex. Mit Moxifloxacin-Hydrochlorid-Lösung (50 mg/mL) mittels Spritze Kanal und Fistelgang durchspült, Kanaleinlage mit Moxifloxacin-Hydrochlorid-Gel (50 mg/mL) und intraligamentale Injektion von Moxifloxacin-Hydrochlorid-Lösung (50 mg/mL). Kanal nur mit Wattebausch verschlossen. Kontrolluntersuchung nach 3 Tagen: Patientin noch nicht völlig beschwerdefrei. Kanal zeigte noch geringfügigen gangränösen Geruch. Wiederholung der Durchspülung mit Moxifloxacin-Hydrochlorid-Lösung (50 mg/mL) und der Kanaleinlage mit Moxifloxacin-Hydrochlorid-Gel (50 mg/mL). Kontrolluntersuchung nach 8 Tagen: Patientin beschwerdefrei. Wurzelfüllung mit Endomethasone. Kontrolluntersuchung nach 3 Wochen: Patientin beschwerdefrei. Abschlußfüllung mit Composite.

**Patient A 3.11** (männlich, 51 Jahre). Klinische Diagnose: Zahn 34, apikale Parodontitis basierend auf Pulpitis purulenta, nicht gangränös, keine Aufbißbeschwerden. Fistel im Wurzelspitzenbereich. Röntgenologisch leichte Aufhellung. Behandlungsverlauf: Kanalaufbereitung bis zum Apex nicht möglich an palatinaler Wurzel. Wurzelkanaleinlage mit Moxifloxacin-Hydrochlorid-Gel (100 mg/mL), provisorischer Verschluß. Kontrolluntersuchung nach 10 Tagen: Patient beschwerdefrei. Wurzelfüllung mit Endomethasone. Patient zur Kontrolluntersuchung nicht erschienen.

**Patient A 3.12** (weiblich, 34 Jahre). Klinische Diagnose: Zahn 44, Fistelbildung, Aufbißschwierigkeiten, gangränös. Behandlungsverlauf: Kanalaufbereitung bis zum Apex. Wurzelkanaleinlage mit Moxifloxacin-Hydrochlorid-Lösung (50 mg/mL) mit Hilfe einer Papierspitze und intraligamentale Injektion von Moxifloxacin-Hydrochlorid-Lösung (50 mg/mL), provisorischer Verschluß. Kontrolluntersuchung nach 8 Tagen: Patientin beschwerdefrei. Wurzelfüllung mit Endomethasone, Abschlußfüllung mit Composite.

**Patient A 3.13** (weiblich, 78 Jahre). Klinische Diagnose: Zahn 15, apikale Parodontitis basierend auf Pulpitis purulenta, leichte Aufbissbeschwerden, nicht gangränös. Behandlungsverlauf: Kanalaufbereitung bis zum Apex, Kanaleinlage mit Moxifloxacin-Hydrochlorid-Lösung (50 mg/mL) mittels Spritze und anschließend Nachfüllen von Moxifloxacin-Hydrochlorid-Gel (50 mg/mL) und intraligamentale Injektion von Moxifloxacin-Hydrochlorid-Lösung (50 mg/mL), provisorischer Verschluss. Kontrolluntersuchung nach 9 Tagen: Patientin beschwerdefrei, Wurzelfüllung mit Endomethasone. Kontrolluntersuchung nach 10 Tagen: Patientin weiterhin beschwerdefrei, Abschlußfüllung Composite.

**Patient A 3.14** (männlich, 30 Jahre). Klinische Diagnose: Zahn 13, gangränös, Aufbissbeschwerden, röntgenologisch kein Befund. Behandlungsverlauf: Kanalaufbereitung bis zum Apex, Kanaleinlage mit Moxifloxacin-Hydrochlorid-Lösung (50 mg/mL) mittels Spritze und anschließend Nachfüllen von Moxifloxacin-Hydrochlorid-Gel (50 mg/mL) und intraligamentale Injektion von Moxifloxacin-Hydrochlorid-Lösung (50 mg/mL), provisorischer Verschluss. Kontrolluntersuchung nach 10 Tagen: Patient beschwerdefrei, Kanal geruchlos, Wurzelfüllung mit Endomethasone.

**Patient A 3.15** (weiblich, 32 Jahre). Klinische Diagnose: Zahn 31, apikale Parodontitis basierend auf Gangrän, Aufbissbeschwerden, röntgenologisch erweiterter Parodontalspalt. Behandlungsverlauf: Kanalaufbereitung bis zum Apex, Kanaleinlage mit Moxifloxacin-Hydrochlorid-Lösung (50 mg/mL) mittels Spritze, anschließend Nachfüllen von Moxifloxacin-Hydrochlorid-Gel (50 mg/mL) und intraligamentale Injektion von Moxifloxacin-Hydrochlorid-Lösung (50 mg/mL), provisorischer Verschluss. Kontrolluntersuchung nach 9 Tagen: Patientin beschwerdefrei, Kanal geruchlos.

**Patient A 3.16** (männlich, 40 Jahre). Klinische Diagnose: Zahn 46, apikale Parodontotis, basierend Pulpitis purulenta, nicht gangränös, röntgenologisch erweiterter Parodontalspalt sichtbar. Behandlungsverlauf: Kanalaufbereitung bis zum Apex, Wurzelkanaleinlage mit Grepafloxacin-Gel (50 mg/mL), provisorischer Verschluss. Kontrolluntersuchung nach 3 Wochen: Patient beschwerdefrei. Wurzelfüllung mit Endomethasone, provisorischer Verschluss. Kontrolluntersuchung nach 1 Woche: Patient weiterhin beschwerdefrei, Abschlussfüllung Composite.

**Patient A 3.17** (weiblich, 33 Jahre). Klinische Diagnose: Zahn 32, apikale Parodontitis basierend auf Gangrän, leichte Aufbissbeschwerden, röntgenologisch schwache Aufhellung sichtbar. Behandlungsverlauf: Kanalaufbereitung bis zum Apex nicht möglich. Wurzelkanaleinlage mit Grepafloxacin-Lösung (20 mg/mL) mittels Spritze, anschließend Nachfüllen von Grepafloxacin-Gel (20 mg/mL) und intraligamentale Injektion von Grepafloxacin-Lösung (50 mg/mL). Kontrolluntersuchung nach 6 Tagen: kein gangränöser Geruch, keine Aufbissbeschwerden. Wurzeifüllung mit N2 medical Kontrolluntersuchung nach 4 Tagen: Patientin beschwerdefrei. Abschlussfüllung mit Composite.

**Patient A 3.18** (weiblich, 55 Jahre). Klinische Diagnose: Zahn 34, apikale Parodontitis basierend auf Gangrän, geringe Aufbissbeschwerden, röntgenologisch schwache Aufhellung sichtbar. Behandlungsverlauf: Kanalaufbereitung bis zum Apex, Wurzelkanaleinlage mit Trovafloxacin-Mesylat-Gel (100 mg/mL) und intraligamentale Injektion von Trovafloxacin-Mesylat-Lösung (50 mg/mL), provisorischer Verschluss. Kontrolluntersuchung nach 12 Tagen: Patientin beschwerdefrei. Wurzelfüllung mit Endomethasone. Kontrolluntersuchung nach 3 Wochen: Patientin weiterhin beschwerdefrei. Abschlussfüllung mit Composite.

**Patient A 3.19** (weiblich, 22 Jahre). Klinische Diagnose: Zahn 11, apikale Parodontitis basierend auf Gangräna simplex. Behandlungsverlauf: Kanalaufbereitung bis zum Apex nicht möglich. Wurzelkanaleinlage mit Trovafloxacin-Mesylat-Lösung (50 mg/mL), provisorischer Verschluss. Kontrolluntersuchung nach 3 Wochen: Patientin beschwerdefrei, Kanal geruchsfrei. Wurzelfüllung mit Endomethasone, Abschlußfüllung mit Glasionomer.

**Patient A 3.20** (weiblich, 77 Jahre). Klinische Diagnose: Zahn 47, apikale Parodontitis basierend auf Pulpitis purulenta, Zahn nicht gangränös, röntgenologisch leicht erweiterter Parodontalspalt an mesialer Wurzel sichtbar. Behandlungsverlauf: Kanäle aufbereitet bis zum Apex. Wurzelkanaleinlage mit Tosufloxacin-Tosylat-Gel (100 mg/mL). Kontrolluntersuchung nach 8 Tagen: Patientin beschwerdefrei. Wurzelfüllung mit Endomethasone, Abschlußfüllung. Patientin zur weiteren Kontrolluntersuchung nicht erschienen.

**Patient A 3.21** (weiblich, 54 Jahre). Klinische Diagnose: Zahn 22, Fistel im Wurzelspitzenbereich, Zahn gangränös, leichte Aufbissbeschwerden. Behandlungsverlauf: Kanalaufbereitung bis zum Apex, Kanal und Fistelgang mit Moxifloxacin-Hydrochlorid-Lösung (50 mg/mL) mittels Spritze durchspült, Kanaleinlage mit Moxifloxacin-Hydrochlorid-Gel (50 mg/mL) und intraligamentale Injektion von Moxifloxacin-Hydrochlorid-Lösung (50 mg/mL). Kanal nur mit Wattebausch verschlossen. Kontrolluntersuchung nach 8 Tagen: Patientin noch nicht völlig beschwerdefrei, Kanal zeigte noch geringfügigen Geruch. Wiederholung der Durchspülung und der Einlage. Kontrolluntersuchung nach 10 Tagen: Patientin beschwerdefrei. Wurzelfüllung mit Endomethasone. Kontrolluntersuchung nach 1 Woche: Patientin beschwerdefrei. Abschlussfüllung mit Composite.

**Patient A 3.22** (männlich, 50 Jahre). Klinische Diagnose: Zahn 35, apikale Parodontitis basierend auf Pulpitis purulenta, nicht gangränös, keine Aufbissbeschwerden. Behandlungsverlauf: Kanalaufbereitung bis zum Apex, Wurzelkanaleinlage mit Tosufloxacin-Tosylat-Gel (50 mg/mL), provisorischer Verschluss.Kontrolluntersuchung nach 10 Tagen: Patient beschwerdefrei. Wurzelfüllung mit Endomethasone. Patient zur Kontrolluntersuchung nicht erschienen.

**Patient A 3.23** (weiblich, 64 Jahre). Klinische Diagnose: Zahn 34, Fistelbildung, Aufbissschwierigkeiten, gangränös. Behandlungsverlauf: Kanalaufbereitung bis zum Apex, Wurzelkanaleinlage mit Grepafloxacin-Lösung (50 mg/mL) mit Hilfe einer Papierspitze, intraligamentale Injektion von Grepafloxacin-Lösung (50 mg/mL), provisorischer Verschluss. Kontrolluntersuchung nach 10 Tagen: Patientin beschwerdefrei. Wurzelfüllung mit Endomethasone, Abschlussfüllung mit Composite.

### Referenzbeispiele B1-B8 Topische Behandlung von Parodontopathien

**Patient B 1** (weiblich, 33 Jahre). Klinische Diagnostik: Zähne 26 und 27, marginale Parodontitis mit freiliegendem Wurzelzement, Taschentiefen von 4 bis 5 mm, starker foetor ex ore. Behandlungsverlauf: Konkremententfernung mittels Ultraschall und manuell, Fadeneinlage mit Moxifloxacin-Hydrochlorid-Gel (50 mg/mL). Abdeckung mit Zahnfleischverband Gingipac. Kontrolluntersuchung nach 3 Tagen: Patientin beschwerdefrei, Gingivalbereiche und Taschen entzündungsfrei.

**Patient B 2** (weiblich, 60 Jahre). Klinische Diagnose: Zähne 14 bis 18, starke marginale Parodontitis mit freiliegendem Wurzelzement, Taschentiefen 5 bis 6 mm. Behandlungsverlauf: Konkremententfernung mit Ultraschall, Fadeneinlage mit Moxifloxacin-Hydrochlorid-Gel (50 mg/mL), Abdeckung mit Zahnfleischverband Gingipac. Kontrolluntersuchung nach 4 Tagen: Patientin beschwerdefrei, Gingivalbereiche und Taschen entzündungsfrei.

**Patient B 3** (männlich, 49 Jahre alt). Klinische Diagnose: Zähne 24 bis 28, marginale Parodontitis mit freiliegendem Wurzelzement, Taschentiefen 4 bis 6 mm. Behandlungsverlauf: Konkremententfernung mit Ultraschall und manuell, Fadeneinlage mit Moxifloxacin-Hydrochlorid (50 mg/mL), Abdeckung mit Zahnfleischverband Gingipac. Kontrolluntersuchung nach 5 Tagen: Patient beschwerdefrei, Gingivalbereiche und Taschen entzündungsfrei.

**Patient B 4** (weiblich, 20 Jahre). Klinische Diagnose: Gesamter Ober- und Unterkieferbereich marginale Parodontitis, starker foetor ex ore, Taschentiefen 4 bis 5 mm. Behandlungsverlauf: Konkremententfernung mit Ultraschall, Medikamentenschiene über 4 Tage für je 10 Minuten mit Moxifloxacin-Hydrochlorid-Gel (0,25 mg/mL) getragen. Kontrolluntersuchung nach 3 Tagen: Patientin beschwerdefrei, keine Entzündungserscheinungen mehr vorhanden.

**Patient B 5** (weiblich, 53 Jahre). Klinische Diagnostik: Zahn 37, marginale Parodontitis mit freiliegendem Wurzelzement, Taschentiefen von 5 mm, starker foetor ex ore. Behandlungsverlauf: Konkremententfernung mittels Ultraschall und manuell, Fadeneinlage mit Moxifloxacin-Hydrochlorid-Gel (50 mg/mL), Abdeckung mit Zahnfleischverband Gingipac. Kontrolluntersuchung nach 8 Tagen: Patientin beschwerdefrei, Gingivalbereiche und Taschen entzündungsfrei.

**Patient B 6** (weiblich, 30 Jahre). Klinische Diagnose: Zähne 25 - 28, starke marginale Parodontitis mit freiliegendem Wurzelzement, Taschentiefen 4 mm. Behandlungsverlauf: Konkremententfernung mit Ultraschall, Fadeneinlage mit Moxifloxacin-Hydrochlorid-Gel (50 mg/mL), intraligamentale Injektion von Moxifloxacin-Hydrochlorid-Lösung (50 mg/mL). Abdeckung mit Zahnfleischverband Gingipac. Kontrolluntersuchung nach 10 Tagen: Patientin beschwerdefrei, Gingivalbereiche und Taschen entzündungsfrei.

**Patient B 7** (männlich, 35 Jahre). Klinische Diagnose: Zähne 14-17, marginale Parodontitis mit freiliegendem Wurzeizement, Taschentiefen 4 mm. Behandlungsverlauf: Konkremententfernung mit Ultraschall und manuell, Fadeneinlage mit Grepafloxacin-Gel (50 mg/mL), intraligamentale Injektion von Grepafloxacin-Lösung (50 mg/mL), Abdeckung mit Zahnfleischverband Gingipac. Kontrolluntersuchung nach 9 Tagen: Patient beschwerdefrei, Gingivalbereiche und Taschen entzündungsfrei.

**Patient B 8** (weiblich, 33 Jahre). Klinische Diagnose: im gesamten Ober- und Unterkieferbereich marginale Parodontitis, starker foetor ex ore, Taschentiefen 3-5 mm. Behandlungsverlauf: Konkremententfernung mit Ultraschall, Medikamentenschiene über 6 Tage für je 10 Minuten mit Trovafloxacin-Mesylat-Gel (0,25 mg/mL) getragen. Kontrolluntersuchung nach 6 Tagen: Patientin beschwerdefrei, keine Entzündungserscheinungen mehr vorhanden.

### Referenzbeispiele C1-C9 Topische Behandlung von Schleimhaut-Knochenwunden

**Patient C 1** (männlich, 14 Jahre). Klinische Diagnose: Zähne 011 und 012, Wundversorgung nach Extraktion, reimplantierte Zähne. Behandlungsverlauf: Röntgenologische Resorption, Ankylose und Sequester. Nach Wundversorgung Excochleation. Applikation eines Moxifloxacin-Hydrochlorid-Geldepots (50 mg/mL) mittels Gazestreifens. Kontrolluntersuchung nach 3 Tagen: Patient beschwerdefrei, gute Wundrandadaptation.

**Patient C 2** (männlich, 16 Jahre). Klinische Diagnose: Regio 36, Wundversorgung nach größeren chirurgischen Eingriffen, leichte submucöse Schwellung, Fistelbildung nach pulpitis purulenta an Zahn 36. Behandlungsverlauf: Incision, Durchspülung mit Moxifloxacin-Hydrochlorid-Lösung (50 mg/mL), Streifeneinlage mit Moxifloxacin-Hydrochlorid-Gel (50 mgmL). Kontrolluntersuchung nach 3 Tagen: Patient beschwerdefrei, guter und schneller Heilungsverlauf.

**Patient C 3** (weiblich, 34 Jahre). Klinische Diagnose: Zähne 26 und 46 mit apikalen Granulomen, starke Aufbißbeschwerden. Behandlungsverlauf: Extraktion im entzündlichen Stadium (Osteotomie), kein Nahtverschluß. Moxifloxacin-Hydrochlorid-Lösung (50 mg/mL), Abdeckung mit Zahnfleischverband Gingipac. Kontrolluntersuchung nach 10 Tagen: Patientin beschwerdefrei, Gingivalbereiche und Taschen entzündungsfrei.

**Patient B 7** (männlich, 35 Jahre). Klinische Diagnose: Zähne 14 - 17, marginale Parodontitis mit freiliegendem Wurzeizement, Taschentiefen 4 mm. Behandlungsverlauf: Konkremententfernung mit Ultraschall und manuell, Fadeneinlage mit Grepafloxacin-Gel (50 mg/mL), intraligamentale Injektion von Grepafloxacin-Lösung (50 mg/mL), Abdeckung mit Zahnfleischverband Gingipac. Kontrolluntersuchung nach 9 Tagen: Patient beschwerdefrei, Gingivalbereiche und Taschen entzündungsfrei.

**Patient B 8** (weiblich, 33 Jahre). Klinische Diagnose: im gesamten Ober- und Unterkieferbereich marginale Parodontitis, starker foetor ex ore, Taschentiefen 3-5 mm. Behandlungsverlauf: Konkremententfernung mit Ultraschall, Medikamentenschiene über 6 Tage für je 10 Minuten mit Trovafloxacin-Mesylat-Gel (0,25 mg/mL) getragen. Kontrolluntersuchung nach 6 Tagen: Patientin beschwerdefrei, keine Entzündungserscheinungen mehr vorhanden.

### C Topische Behandlung von Schleimhaut-Knochenwunden

**Patient C 1** (männlich, 14 Jahre). Klinische Diagnose: Zähne 011 und 012, Wundversorgung nach Extraktion, reimplantierte Zähne. Behandlungsverlauf: Röntgenologische Resorption, Ankylose und Sequester. Nach Wundversorgung Excochleation. Applikation eines Moxifloxacin-Hydrochlorid-Geldepots (50 mg/mL) mittels Gazestreifens. Kontrolluntersuchung nach 3 Tagen: Patient beschwerdefrei, gute Wundrandadaptation.

**Patient C 2** (männlich, 16 Jahre). Klinische Diagnose: Regio 36, Wundversorgung nach größeren chirurgischen Eingriffen, leichte submucöse Schwellung, Fistelbildung nach pulpitis purulenta an Zahn 36. Behandlungsverlauf: Incision, Durchspülung mit Moxifloxacin-Hydrochlorid-Lösung (50 mg/mL), Streifeneinlage mit Moxifloxacin-Hydrochlorid-Gel (50 mgmL). Kontrolluntersuchung nach 3 Tagen: Patient beschwerdefrei, guter und schneller Heilungsverlauf.

**Patient C 3** (weiblich, 34 Jahre). Klinische Diagnose: Zähne 26 und 46 mit apikalen Granulomen, starke Aufbißbeschwerden. Behandlungsverlauf: Extraktion im entzündlichen Stadium (Osteotomie), kein Nahtverschluß. Wundversorgung mit Gaze, getränkt mit Moxifloxacin-Hydrochlorid-Gel (50 mg/mL). Kontrolluntersuchung nach 2 Tagen: Patientin beschwerdefrei.

**Patient C 4** (weiblich, 34 Jahre). Klinische Diagnose: Zahn 48, impaktiert und verlagert. Behandlungsverlauf: Wundversorgung nach größerem chirurgischen Eingriff (Osteotomie), Wundversorgung mit Gaze, getränkt mit Moxifloxacin-Hydrochlorid-Gel (50 mg/mL). Wundverschluß mit zwei Knopfnähten. Kontrolluntersuchung nach 2 Tagen: Leichte Wundschmerzen, kein postoperatives Ödem. Kontrolluntersuchung nach einer Woche: Nahtentfemung, gute Wundrandadaptation, Patientin beschwerdefrei

**Patient C 5** (männlich, 52 Jahre). Klinische Diagnose: Zahn 34, Pulpitis purulenta, Fistelbildung, submucöse Schwellung. Behandlungsverlauf: Trepanation, Incision, Durchspülung von Wurzelkanal und Fistelgang mit Moxifloxacin-Hydrochlorid-Lösung (50 mg/mL), Kanaleinlage mit Moxifloxacin-Hydrochlorid-Gel (50 mg/mL), Abschluß nur mit Wattebausch, Einlage von Gaze in incidiertem Bereich, getränkt mit Moxifloxacin-Hydrochlorid-Gel (50 mg/mL), kein Nahtverschluß. Kontrolluntersuchung nach 3 Tagen: Guter Heilungsverlauf, Patient beschwerdefrei. Wurzelkanalfüllung mit Endomethasone. Kontrolluntersuchung nach 3 Wochen: Patient beschwerdefrei.

**Patient C 6** (männlich, 44 Jahre). Klinische Diagnose: Zahn 36, Wundversorgung nach Extraktion. Behandlungsverlauf: Dolor post, Auffrischung der Alveole, Applikation eines Depots aus Moxifloxacin-Hydrochlorid-Gel (50 mg/mL) mittels Gazestreifen. Kontrolluntersuchung nach 3 Tagen: Patient beschwerdefrei, gute Wundrandadaptation.

**Patient C 7** (männlich, 36 Jahre). Klinische Diagnostik: Zahn 22, Wundversorgung nach Wurzelspitzenresektion auf Grund einer Zyste. Behandlungsverlauf: Incision, Streifeneinlage mit Moxifloxacin-Hydrochlorid-Gel (50 mg/mL). Kontrolluntersuchung nach 3 Tagen: Patient beschwerdefrei, guter Heilungsverlauf.

**Patient C 8** (weiblich, 31 Jahre). Klinische Diagnostik:Zähne 26 und 26 mit apikalen Granulomen, Aufbissbeschwerden. Behandlungsverlauf: Extraktion im entzündlichen Stadium (Osteotomie), Wundversorgung mit Gaze, getränkt mit Grepafloxacin-Gel (50 mg/mL). Kontrolluntersuchung nach 3 Tagen: Patientin ist beschwerdefrei.

**Patient C 9** (männlich, 22 Jahre). Klinische Diagnostik: Zahn 14, Pulpitis purulenta, Fistelbildung, submucöse Schwellung. Behnadlungsverlauf: Trepanation, Incision.Durchspülung von Wurzelkanal und Fistelgang mit Moxifloxacin-Hydrochlorid-Lösung (25 mg/mL), Kanaleinlage mit Grepafloxacin-Gel (25 mg/mL), Abschluss nur mit Wattebausch. Einlage von Gaze in incidiertem Bereich, getränkt mit Moxifloxacin-Hydrochlorid-Gel (25 mg/mL), kein Nahtverschluß. Kontrolluntersuchung nach 6 Tagen: Patient beschwerdefrei. Wurzelkanalfüllung mit Endomethasone. Kontrolluntersuchung nach 2 Wochen: Patient beschwerdefrei.

### D Wundversorgung

**Patient D 1** (männlich, 33 Jahre). Diagnose: Combustio escharotica am linken Unterarm. Behandlungsverlauf: Wundhygiene, Auftragen von Moxifloxacin-Hydrochlorid-Gel (1 %-ig), Wundabdeckung. Regenierierung des Hautepithels mit vollständiger Schließung der Wunde nach einer Woche.

**Patient D 2** (männlich, 25 Jahre). Diagnose: Panaritium parunguale am rechten Mittelfinger. Behandlungsverlauf: Leitungsanästhesie, Eröffnung am Nagelrand, lokale Antibiose mit Moxifloxacin-Hydrochlorid-Gel (1 %-ig), Einlage einer Gummilasche. Patient beschwerdefrei.

**Patient D 3** (männlich, 70 Jahre). Diagnose: Diabetes mellitus, Krankenhausaufenthalt wegen schwerer diabetischer Mikroangiopathie der Füße mit diabetischem Fußsyndrom. Danach ambulante Versorgung des Fußes. Befund: a) tiefes, 2 x 1,5 cm großes Ulcus unter rechter Großzehe, schmierig; b) großes Ulcus D III, 5 mm Durchmesser; c) großer Riss D IV / D V; starke Verhornung des ganzen Fußes. Behandlungsverlauf: systemischer Behandlungsversuch mit Avalox 400 und Clont 400 ohne nennenswerte Befundänderung. Danach Applikation von Verbänden mit Moxifloxacin-Hydrochlorid-Gel (1 %-ig) in wöchentlichem Abstand. Wundkontrollen nach 1. Woche: a) UIcus D I 1 x 0,5 cm, sauber; b) Ulcus D III sauber; c) Ulcus D IV / D V verkrustet. Wundkontrollen nach 2. Woche: a) Ulcus D I stecknadelkopfgroß, sauber; b) Ulcus D III sauberes Granulationsgewebe; c) Ulcus C IV / DV abgeheilt. Wundkontrollen nach 3. Woche: a) Ulcus DI unverändert; b) Ulcus D III stecknadelkopfgroß. Wundkontrollen nach 4. Woche: a) Ulcus D I abgeheilt; b) Ulcus D III abgeheilt.

**Patient D 4** (männlich, 62 Jahre). Diagnose: Diabetes mellitus, diabetische Mikroangiopathie des rechten Fußes mit diabetischem Fußsyndrom. Befund: 1 x 1 cm großes Ulcus, schmierig; starke Verhornung des ganzen Fußes. Behandlungsverlauf: Applikation von Verbänden mit Moxifloxacin-Hydrochlorid-Gel (1 %-ig) in wöchentlichem Abstand. Wundkontrolle nach 1. Woche: Ulcus 0,2 x 0,4 cm, sauber. Wundkontrolle nach 2. Woche: Ulcus abgeheilt.

**Patient D 5** (weiblich, 67 Jahre). Diagnose: diabetische Gangrän an der linken Großzehe. Behandlungsverlauf: Wundhygiene, Abtragen von Hyperkeratosen und Nekrosen. Viermaliges Auftragen von Moxifloxacin-Hydrochlorid-Gel (1 %-ig) in wöchentlichem Abstand, jeweils Wundabdeckung. Regenierierung des Hautepithels mit vollständiger Schließung der Wunde, Revaskularisierung.

**Patient D 6** (männlich, 34 Jahre). Diagnose: Erysipel am rechten Unterschenkel. Behandlungsverlauf: Ruhigstellung, lokale Antibiose mit Moxifloxacin-Hydrochlorid-Gel (1 %-ig), lokale Umschläge. Nach 2 Tagen Rezidivprophylaxe. Patient beschwerdefrei.

**Patient D 7** (weiblich, 52 Jahre). Diagnose: Phlegmone an linker Innenhand. Behandlungsverlauf: Incision und breite Eröffnung der betroffenen Gewebsareale, lokale Antibiose mit Moxifloxacin-Hydrochlorid-Gel (1 %-ig), Wundversorgung. Nach 2 Tagen Wiederholung der lokalen Antibiose. Patient beschwerdefrei.

**Patient D 8** (weiblich, 28 Jahre). Diagnose: Furunkel an linkem Unterarm. Behandlungsverlauf: Incision und offene Wundbehandlung mit Moxifloxacin-Hydrochlorid-Lösung (1 %-ig). Patient beschwerdefrei.

**Patient D 9** (weiblich, 44 Jahre). Diagnose: Karbunkel im Nacken. Behandlungsverlauf: Ausschneiden aller nekrotischen Areale, offene Wundbehandlung mit Moxifloxacin-Hydrochlorid-getränktem Umschlag. Patient beschwerdefrei.

## Patentansprüche

1. Verwendung wenigstens einer Verbindung der allgemeinen Formel (I) 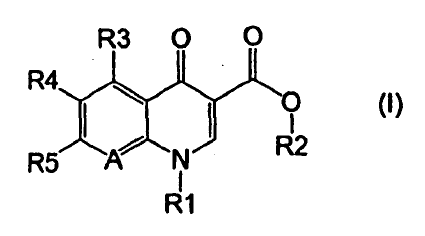 in welcher
A für CH, CCI, CBr, C-CH₃, C-CN, C-OCH₃, C-OCHF₂ oder N steht,
R1 für Cycloalkyl, Bicycloalkyl, oder 2- Fluorcyclopropyl, steht,
R2 für Wasserstoff, Methyl oder Ethyl steht,
R3 für Wasserstoff, Halogen, Methyl, Amino oder NH-NH₂ steht,
R4 für Wasserstoff, Halogen oder Amino steht,
R5 für einen gegebenenfalls einfach oder mehrfach substituierten gesättigten oder wenigstens eine Doppelbindung aufweisenden mono-, bi- odertricyclischen Alicyclus mit gegebenenfalls wenigstens einem Heteroatom im Ringsystem oder einen gegebenenfalls wenigstens ein Heteroatom aufweisenden aromatischen Mono-, Bi- oder Tricyclus steht,
und/oder ihres entsprechenden Hydrates und/oder ihres entsprechenden physiologisch verträglichen Säureadditionssalzes und/oder ggf. ihres entsprechenden physiologisch verträglichen Salzes der zugrundeliegenden Carbonsäure, worin R2 für H steht und/oder entsprechender Enantiomere und/oder entsprechender Diastereomere und/oder des entsprechenden Racemates und/oder eines entsprechenden Gemisches aus wenigstens zwei der vorstehend genannten Verbindungen sowie gegebenenfalls weiterer physiologisch verträglicher Hilfsstoffe zur Herstellung eines Arzneimittels zur topischen und/oder lokalen Behandlung von Wundformen bei Infektionen, vorzugsweise bei postoperativen oder posttraumatischen Wundinfektionen, vorzugsweise postraumatischen Wundinfektionen hervorgerufen durch Schnitte, Quetschungen, Bisse oder Schüsse, infizierten Verbrennungen, Handinsektionen, vorzugsweise Panaritium cutaneum, Panaritium subcutaneum, Panaritium ossale, Panaritium articulare oderTendovaginitis purulenta, postoperativer Sepsis, infizierten Ulzera, Gangränen, Schleimhautulzerationen, akuten bakteriellen Hautinfektionen, vorzugsweise Pyrodermien, Erysipeln, Furunkeln, Karbunkeln, Phlegmonen, Abszessen, Ulcus cruris, infizierter Dekubitalulzera, hämorrhagischen Blasen, Erysipeloiden oder Erythrasma, und/oder chronischen bakteriellen Hautinfektionen, vorzugsweise Lupus vulgaris, Schwimmbad-Granulomen, Buruli-Ulkus oder Aktinomykose, sekundär infizierten Dermatosen, Akne oder Rosazea.

2. Verwendung gemäß Anspruch 1, **dadurch gekennzeichnet, dass** in der allgemeinen Formel (1):
A für CH, CCI, CBr, C-CH₃, C-CN, C-OCH₃, C-OCHF₂ oder N steht,
R1 für Cyclopropyl, Bicyclo(1.1.1) pent-1-yl oder, Fluorcyclopropyl steht,
R2 für Wasserstoff, Methyl oder Ethyl steht,
R3 für Wasserstoff, F, CI, Br, Methyl, Amino oder NH-NH₂ steht,
R4 für Wasserstoff, F oder Amino steht,
R5 für gegebenenfalls einfach oder mehrfach substituiertes Cyclopropyl, Azetidinyl, Pyrrolidinyl, Piperidinyl, Piperazinyl, Morpholinyl, Phenyl, Pyrrolyl, Pyridyl oder Imidazolyl steht, wobei gegebenenfalls auch wenigstens zwei Substituenten miteinander verknüpft sein können.

3. Verwendung nach Anspruch 1 oder 2, **dadurch gekennzeichnet, dass** in der allgemeinen Formel (I):
A für CH, CCI, C-CN, C-OCH₃ oder N steht,
R1 für Cyclopropyl oder, 2-Fluorcyclopropyl steht,
R2 für Wasserstoff steht,
R3 für Wasserstoff oder Amino steht,
R4 für Wasserstoff oder F steht,
R5 für gegebenenfalls einfach oder mehrfach substituiertes Pyrrolidinyl, für gegebenenfalls einfach oder mehrfach substituiertes Pyrrolidinyl, Piperidinyl, Piperazinyl oder Morpholinyl steht, wobei gegebenenfalls auch wenigstens zwei Substituenten miteinander verknüpft sein können.

4. Verwendung nach einem der Ansprüche 1 bis 3, **dadurch gekennzeichnet, dass** in der allgemeinen Formel (I):
A für CH, CCI, C-OCH₃ oder N steht,
R1 für Cyclopropyl steht,
R2 für Wasserstoff steht,
R3 für Wasserstoff oder Amino steht,
R4 für Wasserstoff oder F steht,
R5 fürgegebenenfalls mit Amino, Methyl, Aminomethyl und/oder Methoxyimino substituiertes Pyrrolidinyl, Piperidinyl, Piperazinyl, Morpholinyl, 3-Azabicyclo(3.1.0)hexyl oder für Piperidino-pyrrolidinyl steht.

5. Verwendung gemäß einem der Ansprüche 1 bis 4, **dadurch gekennzeichnet, dass**
- 1-Cyclopropyl-6-fluor-1,4-dihydro-8-methoxy-7-[3-(methylamino)-1-piperidinyl]-4-oxo-3-chinolincarbonsäure (Balofloxacin),
- 8-Chlor-1-cyclopropyl-6-fluor-1.4-dihydro-7-[(4αS,7αS)-octahydro-6H-pyrrolo[3.4-b]pyridin-6-yl]-4-oxo-3-chinolincarbonsäure Monohydrochlorid (BAY Y3118),
- 1-Cyclopropyl-6-fluor-8-difluormethoxy-1,4-dihydro-7-((3S)-methyl-1-piperazinyl)-4-oxo-3-chinolincarbonsäure Hydrochlorid (Caderofloxacin),
- 1 1-Cyctopropyl-6-fluor-1,4-dihydro-4-oxo-7-(1-piperazinyl)-3-chinolincarbonsäure (Ciprofloxacin),
- 7-(3-Amino-1 -pyrrolidinyl)-8-chlor-1 -cyclopropyl-6-fluor-1,4-dihydro-4-oxo-3-chinolincarbonsäure (Clinafloxacin),
- (1α,5α,6β)-(+)-(6-Amino-1-methyl-3-azabicyclo[3.2.0]hept-3-yl)-1-cyclopropyl-6-fluor-1,4-dihydro-4-oxo-1,8-naphthyridin-3-carbonsäure (Ecenofloxacin),
- 1-Cyclopropyl-7-(4-ethyl-1-piperazinyl)-6-fluor-1,4-dihydro-4-oxo-3chinolincarbonsäure (Enrofloxacin),
- 1-Cyclopropyl-6-fluor-1,4-dihydro-8-methoxy-7-(3-methyl-1-piperazinyl)-4-oxo-3-chinolincarbonsäure (Gatifloxacin),
- 7-[(4Z)-3-(Aminomethyl)-4-(methoxyimino)-1-pyrrolidinyl]-1-cyclopropyl-6-fluor-1,4-dihydro-4-oxo-1,8-naphthyridin-3-carbonsäure (Gemifloxacin),
- 1-Cyclopropyl-6-fluor-1,4-dihydro-5-methyl-7-(3-methyl-1-piperazinyl)-4-oxo-3-chinolincarbonsäure (Grepafloxacin),
- 1 -Cyclopropyl-6-fluor-1,4-dihydro-8-methoxy-7-[(4aS.7aS)-octahydro-6H-pyrrolo[3,4-b]pyridin-6yl]-4-oxo-3-chinolincarbonsäure (Moxifloxacin),
- 5-Amino-7-[(7S)-7-amino-5-azaspiro[2.4]hept-5-yl]-1-cyclopropyl-6-fluor-1,4-dihydro-8-methyl-4-oxo-3-chinolincarbonsäure (Olamufloxacin),
- 7-[(7S)-7-Amino-5-azaspiro[2.4]hept-5-yl]-8-chloro-6-fluor-1-[(1R,2S)-2-fluorcyclopropyl]-1.4-dihydro-4-oxo-3-chinolincarbonsäure (Sitafloxacin),
oder ein Gemisch aus wenigstens zwei dieser Verbindungen eingesetzt wird.

6. Verwendung gemäß Anspruch 5, **dadurch gekennzeichnet, dass**
- 7-(3-Amino-1-pyrrolidinyl)-8-chlor-1-cyclopropyl-6-fluor-1,4-dihydro-4-oxo-3-chlnolincarbonsäure (Clinafloxacin),
- 1-Cydopropyl-6-fluor-1,4-dihydro-8-methoxy-7-(3-methyl-1-piperazinyl)-4-oxo-3-chinolincarbonsäure (Gatifloxacin),
- 7-[(4Z)-3-(Aminomethyl)-4-(methoxyimino)-1-pyrrotidinyl]-1-cyclopropyl-6-fluor-1,4-dihydro-4-oxo-1,8-naphthyridin-3-carbonsäure (Gemifloxacin),
- 1-Cyclopropyl-6-fluor-1,4-dihydro-8-methoxy-7-[(4aS,7aS)-octahydro-6H-pyrrolo[3,4-b]pyridin-6-yl]-4-oxo-3-chinolincarbonsäure (Moxifloxacin),
oder ein Gemisch aus wenigstens zwei dieser Verbindungen eingesetzt wird.

7. Verwendung gemäß Anspruch 5 oder 6, **dadurch gekennzeichnet, dass**
- 1-CyGopropyl-6-fluor-1,4-dihydro-8-methoxy-7-(3-methyl-1-piperazinyl)-4-oxo-3-chinolincarbonsäure (Gatifloxacin),
- 7-[(4Z)3-(Aminomethyl)-4-(methoxyimino)-1-pyrrolidinyl]-1-cyclopropyl-6-fluor-1,4-dihydro-4-oxo-1,8-naphthyridin-3-carbonsäure (Gemifloxacin) oder
- 1-Cyclopropyl-6-fluor-1,4-dihydro-8-methoxy-7-[(4aS,7aS)-octahydro-6H-pyrrolo[3,4-b]pyridin-6-yl]-4-oxo-3-Chinolincarbonsäure (Moxifloxacin)
oder ein Gemisch aus wenigstens zwei dieser Verbindungen eingesetzt wird.

8. Verwendung gemäß einem der Ansprüche 1 bis 7, **dadurch gekennzeichnet, dass** die Säureadditionssalze ausgewählt sind aus der Gruppe Hydrochlorid, Hydrobromid, Methansulfonat und Tolylsulfonat.

9. Verwendung gemäß einem der Ansprüche 1 bis 8, **dadurch gekennzeichnet, dass** die physiologisch verträglichen Salze der Carbonsäure ausgewählt sind aus der Gruppe der Alkalisalze, Erdalkalisalze, Ammoniumsalze, Silbersalze und Guanidiniumsalze.

10. Verwendung gemäß einem der Ansprüche 1 bis 9, **dadurch gekennzeichnet, dass** das Arzneimittel zur Behandlung von Mensch oder Tier eingesetzt wird.

11. Verwendung gemäß einem der Ansprüche 1 bis 10, **dadurch gekennzeichnet, dass** das Arzneimittel in Form einer Lösung, einer Suspension, einer Emulsion, in Form von Liposomen oder in Form von Mizellen vorliegt und gegebenenfalls auf ein Trägermaterial aufgebracht oder darin eingebracht worden ist.

12. Verwendung gemäß Anspruch 11, **dadurch gekennzeichnet, dass** das Arzneimittel in Form einer wässrigen Lösung vorliegt.

13. Verwendung gemäß Anspruch 11 oder 12, **dadurch gekennzeichnet, dass** das Arzneimittel als weitere physiologisch verträgliche Hilfsstoffe Lösungsmittel, Löslichkeitsverbesserer, Verdickungsmittel, Lösungsvermittler, Solubilisatoren, Konservierungsmittel, Emulgatoren, Schleimstoffe, Osmolalitätsregulatoren, Antioxidantien, Chelatbildner, Desinfektionsmittel, Dispergiermittel, Emulsionsstabilisatoren, Hydrokolloide, Netzmittel oder ein Gemisch aus wenigstens zwei der vorstehend genannten Hilfsstoffe enthält.

## Claims

1. Use of at least one compound of the general formula (I) 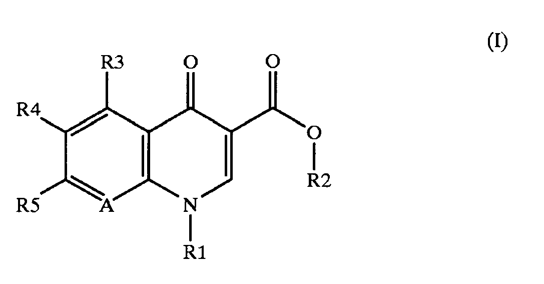 in which
A is CH, CCl, CBr, C-CH₃, C-CN, C-OCH₃, C-OCHF₂ or N,
R1 is cycloalkyl, bicycloalkyl or 2-fluorocyclopropyl,
R2 is hydrogen, methyl or ethyl,
R3 is hydrogen, halogen, methyl, amino or NH-NH2,
R4 is hydrogen, halogen or amino,
R5 is an optionally monosubstituted or poly- substituted mono-, bi- or tricyclic alicycle which is saturated or has at least one double bond and which optionally has at least one heteroatom in the ring system, or an aromatic mono-, bi- or tricycle optionally having at least one heteroatom,
and/or its corresponding hydrate and/or its corresponding physiologically compatible acid addition salt and/or optionally its corresponding physiologically compatible salt of the parent carboxylic acid, in which R2 is H and/or corresponding enantiomers and/or corresponding diastereomers and/or the corresponding racemate and/or a corresponding mixture of at least two of the abovementioned compounds, and optionally other physiologically compatible auxiliary substances
for the preparation of a medicament for the topical and/or local treatment of wound forms in the case of infections, preferably in the case of post-operative or post-traumatic wound infections, preferably post-traumatic wound infections brought about by cuts, contusions, bites or gunshots, infected burns, hand infections, preferably panaritium cutaneum, panaritium subcutaneum, panaritium ossale, panaritium articulare or tendovaginitis purulenta, post-operative sepsis, infected ulcers, gangrenes, mucosal ulcerations, acute bacterial skin infections, preferably pyrodermas, erysipelas, furuncles, carbuncles, phlegmons, abscesses, ulcer cruris, infected decubital ulcers, blood blisters, erysipeloids or erythrasma, and/or chronic bacterial skin infections, preferably lupus vulgaris, swimming-pool granulomas, Buruli ulcers or actinomycosis, secondarily infected dermatoses, acne or rosacea.

2. Use according to Claim 1, **characterized in that** in the general formula (I):
A is CH, CCl, CBr, C-CH₃, C-CN, C-OCH₃, C-OCHF₂ or N,
R1 is cyclopropyl, bicyclo[1.1.1]pent-1-yl or 2- fluorocyclopropyl,
R2 is hydrogen, methyl or ethyl,
R3 is hydrogen, F, Cl, Br, methyl, amino or NH-NH₂,
R4 is hydrogen, F or amino,
R5 is optionally monosubstituted or polysubstituted cyclopropyl, azetidinyl, pyrrolidinyl, piperidinyl, piperazinyl, morpholinyl, phenyl, pyrrolyl, pyridyl or imidazolyl, it also being possible optionally for at least two substituents to be coupled together.

3. Use according to Claim 1 or 2, **characterized in that** in the general formula (I):
A is CH, CCl, C-CN, C-OCH₃ or N,
R1 is cyclopropyl or 2-fluorocyclopropyl,
R2 is hydrogen,
R3 is hydrogen or amino,
R4 is hydrogen or F,
R5 is optionally monosubstituted or polysubstituted pyrrolidinyl, piperidinyl, piperazinyl or morpholinyl, it also being possible optionally for at least two substituents to be coupled together.

4. Use according to one of Claims 1 to 3, **characterized in that** in the general formula (I):
A is CH, CCl, C-OCH₃ or N,
R1 is cyclopropyl,
R2 is hydrogen,
R3 is hydrogen or amino,
R4 is hydrogen or F,
R5 is optionally amino-, methyl-, aminomethyl- and/or methoxyimino-substituted pyrrolidinyl, piper- idinyl, piperazinyl, morpholinyl, 3- azabicyclo[3.1.0]hexyl or is piperidino- pyrrolidinyl.

5. Use according to one of Claims 1 to 4, **characterized in that**
- 1-cyclopropyl-6-fluoro-1,4-dihydro-8-methoxy-7-[3-(methylamino)-1-piperidinyl]-4-oxo-3-quinolinecarboxylic acid (balofloxacin),
- 8-chloro-1-cyclopropyl-6-fluoro-1,4-dihydro-7-[(4αS,7αS)-octahydro-6H-pyrrolo[3,4-b]pyridin-6-yl]-4-oxo-3-quinolinecarboxylic acid monohydrochloride (BAY Y3118),
- 1-cyclopropyl-6-fluoro-8-difluoromethoxy-1,4-dihydro-7-((3S)-methyl-1-piperazinyl)-4-oxo-3-quinolinecarboxylic acid hydrochloride (caderofloxacin),
- 1-cyclopropyl-6-fluoro-1,4-dihydro-4-oxo-7-(1-piperazinyl)-3-quinolinecarboxylic acid (ciprofloxacin)
- 7-(3-amino-1-pyrrolidinyl)-8-chloro-1-cyclopropyl-6-fluoro-1,4-dihydro-4-oxo-3-quinolinecarboxylic acid (clinafloxacin),
- (1α,5α,6β)-(+)-7-(6-amino-1-methyl-3-azabicyclo-[3.2.0]hept-3-yl)-1-cyclopropyl-6-fluoro-1,4-dihydro-4-oxo-1,8-naphthyridine-3-carboxylic acid (ecenofloxacin),
- 1-cyclopropyl-7-(4-ethyl-1-piperazinyl)-6-fluoro-1,4-dihydro-4-oxo-3-quinolinecarboxylic acid (enrofloxacin),
- 1-cyclopropyl-6-fluoro-1,4-dihydro-8-methoxy-7-(3-methyl-1-piperazinyl)-4-oxo-3-quinolinecarboxylic acid (gatifloxacin),
- 7-[(4Z)-3-(aminomethyl)-4-(methoxyimino)-1-pyrrolidinyl]-1-cyclopropyl-6-fluoro-1,4-dihydro-4-oxo-1,8-naphthyridine-3-carboxylic acid (gemifloxacin),
- 1-cyclopropyl-6-fluoro-1,4-dihydro-5-methyl-7-(3-methyl-1-piperazinyl)-4-oxo-3-quinolinecarboxylic acid (grepafloxacin),
- 1-cyclopropyl-6-fluoro-1,4-dihydro-8-methoxy-7-[(4aS,7aS)-octahydro-6H-pyrrolo[3,4-b]pyridin-6-yl]-4-oxo-3-quinolinecarboxylic acid (moxifloxacin),
- 5-amino-7-[(7S)-7-amino-5-azaspiro[2.4]hept-5-yl]-1-cyclopropyl-6-fluoro-1,4-dihydro-8-methyl-4-oxo-3-quinolinecarboxylic acid (olamufloxacin),
- 7-[(7S)-7-amino-5-azaspiro[2.4]hept-5-yl]-8-chloro-6-fluoro-1-[(1R,2S)-2-fluorocyclopropyl]-1,4-dihydro-4-oxo-3-quinolinecarboxylic acid (sitafloxacin),
or a mixture of at least two of these compounds, is used.

6. Use according to Claim 5, **characterized in that**
- 7-(3-amino-1-pyrrolidinyl)-8-chloro-1-cyclopropyl-6-fluoro-1,4-dihydro-4-oxo-3-quinolinecarboxylic acid (clinafloxacin),
- 1-cyclopropyl-6-fluoro-1,4-dihydro-8-methoxy-7-(3-methyl-1-piperazinyl)-4-oxo-3-quinolinecarboxylic acid (gatifloxacin),
- 7-[(4Z)-3-(aminomethyl)-4-(methoxyimino)-1-pyrrolidinyl]-1-cyclopropyl-6-fluoro-1,4-dihydro-4-oxo-1,8-naphthyridine-3-carboxylic acid (gemifloxacin),
- 1-cyclopropyl-6-fluoro-1,4-dihydro-8-methoxy-7-[(4aS,7aS)-octahydro-6H-pyrrolo[3,4-b]pyridin-6-yl]-4-oxo-3-quinolinecarboxylic acid (moxifloxacin),
or a mixture of at least two of these compounds, is used.

7. Use according to Claim 5 or 6, **characterized in that**
- 1-cyclopropyl-6-fluoro-1,4-dihydro-8-methoxy-7-(3-methyl-1-piperazinyl)-4-oxo-3-quinolinecarboxylic acid (gatifloxacin),
- 7-[(4Z)-3-(aminomethyl)-4-(methoxyimino)-1-pyrrolidinyl]-1-cyclopropyl-6-fluoro-1,4-dihydro-4-oxo-1,8-naphthyridine-3-carboxylic acid (gemifloxacin) or
- 1-cyclopropyl-6-fluoro-1,4-dihydro-8-methoxy-7-[(4aS,7aS)-octahydro-6H-pyrrolo[3,4-b]pyridin-6-yl]-4-oxo-3-quinolinecarboxylic acid (moxifloxacin),
or a mixture of at least two of these compounds, is used.

8. Use according to one of Claims 1 to 7, **characterized in that** the acid addition salts are selected from the group consisting of hydrochloride, hydrobromide, methanesulphonate and tolylsulphonate.

9. Use according to one of Claims 1 to 8, **characterized in that** the physiologically compatible salts of the carboxylic acid are selected from the group consisting of alkali metal salts, alkaline earth metal salts, ammonium salts, silver salts and guanidinium salts.

10. Use according to one of Claims 1 to 9, **characterized in that** the medicament is used for the treatment of humans or animals.

11. Use according to one of Claims 1 to 10, **characterized in that** the medicament is in the form of a solution, a suspension, an emulsion, in the form of liposomes or in the form of micelles and has optionally been applied to or incorporated in a carrier material.

12. Use according to Claim 11, **characterized in that** the medicament is in the form of an aqueous solution.

13. Use according to Claim 11 or 12, **characterized in that** the medicament comprises, as other physiologically compatible auxiliary substances, solvents, solubility improvers, thickeners, solubilizers, preservatives, emulsifiers, mucins, osmolality regulators, antioxidants, chelating agents, disinfectants, dispersants, emulsion stabilizers, hydrocolloids, wetting agents or a mixture of at least two of the abovementioned auxiliary substances.

## Revendications

1. Utilisation d'au moins un composé de formule générale (I), 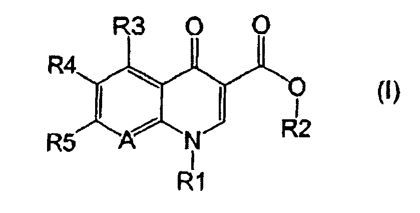 dans laquelle
A représente CH, CCl, CBr, C-CH₃, C-CN, C-OCH₃, C- OCHF₂ ou N,
R1 représente cycloalkyle, bicycloalkyle ou 2- fluorocyclopropyle,
R2 représente hydrogène, méthyle ou éthyle,
R3 représente hydrogène, halogène, méthyle, amino ou NH-NH₂,
R4 représente hydrogène, halogène, ou amino,
R5 représente un alicycle monocyclique, bicyclique ou tricyclique, le cas échéant monosubstitué ou polysubstitué, saturé ou présentant au moins une double liaison, comprenant le cas échéant au moins un hétéroatome dans le système cyclique ou un monocycle, un bicycle ou un tricycle aromatique présentant le cas échéant au moins un hétéroatome,
et/ ou leur hydrate correspondant et/ou leur sel d'addition d'acide physiologiquement acceptable et/ou le cas échéant leur sel physiologiquement acceptable correspondant de l'acide carboxylique de base, R2 représentant H et/ou les énantiomères correspondants et/ou les diastéréo-isomères correspondants et/ou du racémate correspondant et/ou d'un mélange correspondant d'au moins deux des composés susmentionnés ainsi que le cas échéant d'autres adjuvants physiologiquement acceptables
pour la préparation d'un médicament destiné au traitement topique et/ou local de formes de plaies en cas d'infections, de préférence en cas d'infections de plaies post-opératoires ou post-traumatiques, de préférence en cas d'infections de plaies post-traumatiques provoquées par des coupures, des contusions, des morsures ou des coups de feu, des brûlures infectées, des infections des mains, de préférence le Panaritium cutaneum, le Panaritium subcutaneum, le Panaritium ossale, le Panaritium articulare ou le Tendovaginitis purulenta, la septicémie post-opératoire, les ulcères infectés, les gangrènes, les ulcérations des muqueuses, les
infections bactériennes aiguës de la peau, de préférence les pyodermies, les érysipèles, les furoncles, les anthrax, les phlegmons, les abcès, les Ulcus cruris, les escarres infectées, les cloches hémorragiques, les érysipéloïdes ou érythrasma, et/ou les infections bactériennes chroniques de la peau, de préférence le Lupus vulgaris, les granulomes de piscine, l'ulcère de Buruli ou l'actinomycose, les dermatoses infectées secondaires, l'acné ou la rosacée.

2. Utilisation selon la revendication 1, **caractérisée en ce que**, dans la formule générale (I):
A représente CH, CCl, CBr, C-CH₃, C-CN, C-OCH₃, C- OCHF₂ ou N,
R1 représente cyclopropyle, bicyclo(1.1.1)pent-1-yle ou 2-fluorocyclopropyle,
R2 représente hydrogène, méthyle ou éthyle,
R3 représente hydrogène, F, Cl, Br, méthyle, amino ou NH-NH₂,
R4 représente hydrogène, F ou amino,
R5 représente cyclopropyle, azétidinyle, pyrrolidinyle, pipéridinyle, pipérazinyle, morpholinyle, phényle, pyrrolyle, pyridyle ou imidazolyle le cas échéant monosubstitués ou polysubstitués, au moins deux substituants pouvant le cas échéant également être liés l'un à l'autre.

3. Utilisation selon la revendication 1 ou 2, **caractérisée en ce que**, dans la formule générale (I):
A représente CH, CCl, C-CN, C-OCH₃ ou N,
R1 représente cyclopropyle ou 2-fluorocyclopropyle,
R2 représente hydrogène,
R3 représente hydrogène ou amino,
R4 représente hydrogène ou F,
R5 représente pyrrolidinyle, pipéridinyle, pipérazinyle ou morpholinyle le cas échéant monosubstitués ou polysubstitués, au moins deux substituants pouvant le cas échéant également être liés l'un à l'autre.

4. Utilisation selon l'une quelconque des revendications 1 à 3, **caractérisée en ce que**, dans la formule générale (I):
A représente CH, CCl, C-OCH₃ ou N,
R1 représente cyclopropyle,
R2 représente hydrogène,
R3 représente hydrogène ou amino,
R4 représente hydrogène ou F,
R5 représente pyrrolidinyle, pipéridinyle, pipérazinyle, morpholinyle, 3- azabicyclo(3.1.0)hexyle le cas échéant substitués par amino, méthyle, aminométhyle et/ou méthoxyimino ou pipéridinopyrrolidinyle.

5. Utilisation selon l'une quelconque des revendications 1 à 4, **caractérisée en ce qu'**on utilise
- l'acide 1-cyclopropyl-6-fluoro-1,4-dihydro-8-méthoxy-7-[3-(méthylamino)-1-pipéridinyl]-4-oxo-3-quinoléinecarboxylique (Balofloxacin),
- le monochlorhydrate de l'acide 8-chloro-1-cyclopropyl-6-fluoro-1,4-dihydro-7-[(4αS,7αS)-octahydro-6H-pyrrolo[3,4-b]pyridin-6-yl]-4-oxo-3-quinoléinecarboxylique (BAY Y3118),
- le chlorhydrate de l'acide 1-cyclopropyl-6-fluoro-8-difluorométhoxy-1,4-dihydro-7-((3S)-méthyl-1-pipérazinyl)-4-oxo-3-quinoléinecarboxylique (Caderofloxacin),
- l'acide 1-cyclopropyl-6-fluoro-1,4-dihydro-4-oxo-7-(1-pipérazinyl)-3-quinoléinecarboxylique (Ciprofloxacin),
- l'acide 7-(3-amino-1-pyrrolidinyl)-8-chloro-1-cyclopropyl-6-fluoro-1,4-dihydro-4-oxo-3-quinoléinecarboxylique (Clinafloxacin),
- l'acide (1α,5α,6β)-(+)-7-(6-amino-1-méthyl-3-azabicyclo[3.2.0]hept-3-yl)-1-cyclopropyl-6-fluoro-1,4-dihydro-4-oxo-1,8-naphtyridine-3-carboxylique (Ecenofloxacin),
- l'acide 1-cyclopropyl-7-(4-éthyl-1-pipérazinyl)-6-fluoro-1,4-dihydro-4-oxo-3-quinoléinecarboxylique (Enrofloxacin),
- l'acide 1-cyclopropyl-6-fluoro-1,4-dihydro-8-méthoxy-7-(3-méthyl-1-pipérazinyl)-4-oxo-3-quinoléinecarboxylique (Gatifloxacin),
- l'acide 7-[(4Z)-3-(aminométhyl)-4-(méthoxyimino)-1-pyrrolidinyl]-1-cyclopropyl-6-fluoro-1,4-dihydro-4-oxo-1,8-naphtyridine-3-carboxylique (Gemifloxacin),
- l'acide 1-cyclopropyl-6-fluoro-1,4-dihydro-5-méthyl-7-(3-méthyl-1-pipérazinyl)-4-oxo-3-quinoléinecarboxylique (Grepafloxacin),
- l'acide 1-cyclopropyl-6-fluoro-1,4-dihydro-8-méthoxy-7-[(4aS,7aS)-octahydro-6H-pyrrolo[3,4-b]pyridin-6-yl]-4-oxo-3-quinoléinecarboxylique (Moxifloxacin),
- l'acide 5-amino-7-[(7S)-7-amino-5-azaspiro[2.4]hept-5-yl]-1-cyclopropyl-6-fluoro-1,4-dihydro-8-méthyl-4-oxo-3-quinoléinecarboxylique (Olamufloxacin),
- l'acide 7-[(7S)-7-amino-5-azaspiro[2.4]hept-5-yl]-8-chloro-6-fluoro-1-[(1R,2S)-2-fluoroyclopropyl]-1,4-dihydro-4-oxo-3-quinoléinecarboxylique (Sitafloxacin),
ou un mélange d'au moins deux de ces composés.

6. Utilisation selon la revendication 5, **caractérisée en ce qu'**on utilise
- l'acide 7-(3-amino-1-pyrrolidinyl)-8-chloro-1-cyclopropyl-6-fluoro-1,4-dihydro-4-oxo-3-quinoléinecarboxylique (Clinafloxacin),
- l'acide 1-cyclopropyl-6-fluoro-1,4-dihydro-8-méthoxy-7-(3-méthyl-1-pipérazinyl)-4-oxo-3-quinoléinecarboxylique (Gatifloxacin),
- l'acide 7-[(4Z)-3-(aminométhyl)-4-(méthoxyimino)-1-pyrrolidinyl]-1-cyclopropyl-6-fluoro-1,4-dihydro-4-oxo-1,8-naphtyridine-3-carboxylique (Gemifloxacin),
- l'acide 1-cyclopropyl-6-fluoro-1,4-dihydro-8-méthoxy-7-[(4aS,7aS)-octahydro-6H-pyrrolo[3,4-b]pyridin-6-yl]-4-oxo-3-quinoléinecarboxylique (Moxifloxacin),
ou un mélange d'au moins deux de ces composés.

7. Utilisation selon la revendication 5 ou 6, **caractérisée en ce qu'**on utilise
- l'acide 1-cyclopropyl-6-fluoro-1,4-dihydro-8-méthoxy-7-(3-méthyl-1-pipérazinyl)-4-oxo-3-quinoléinecarboxylique (Gatifloxacin),
- l'acide 7-[(4Z)-3-(aminométhyl)-4-(méthoxyimino)-1-pyrrolidinyl]-1-cyclopropyl-6-fluoro-1,4-dihydro-4-oxo-1,8-naphtyridine-3-carboxylique (Gemifloxacin) ou
- l'acide 1-cyclopropyl-6-fluoro-1,4-dihydro-8-méthoxy-7-[(4aS,7aS)-octahydro-6H-pyrrolo[3,4-b]pyridin-6-yl]-4-oxo-3-quinoléinecarboxylique (Moxifloxacin)
ou un mélange d'au moins deux de ces composés.

8. Utilisation selon l'une quelconque des revendications 1 à 7, **caractérisée en ce que** les sels d'addition d'acide sont choisis dans le groupe formé par le chlorhydrate, le bromhydrate, le méthanesulfonate et le toluylsulfonate.

9. Utilisation selon l'une quelconque des revendications 1 à 8, **caractérisée en ce que** les sels physiologiquement acceptables de l'acide carboxylique sont choisis dans le groupe des sels de métal alcalin, alcalino-terreux, d'ammonium, d'argent et de guanidinium.

10. Utilisation selon l'une quelconque des revendications 1 à 9, **caractérisée en ce que** le médicament est utilisé pour le traitement de l'homme ou de l'animal.

11. Utilisation selon l'une quelconque des revendications 1 à 10, **caractérisée en ce que** le médicament se trouve sous forme d'une solution, d'une suspension, d'une émulsion, sous forme de liposomes ou sous forme de micelles et est le cas échéant appliqué sur ou incorporé dans un matériau support.

12. Utilisation selon la revendication 11, **caractérisée en ce que** le médicament se trouve sous forme d'une solution aqueuse.

13. Utilisation selon la revendication 11 ou 12, **caractérisée en ce que** le médicament contient comme autres adjuvants physiologiquement acceptables des solvants, des agents améliorant la solubilité, des épaississants, des promoteurs de solubilisation, des solubilisateurs, des conservateurs, des émulsifiants, des mucilages, des régulateurs de l'osmolalité, des antioxydants, des agents de formation de chélates, des désinfectants, des dispersants, des stabilisations d'émulsion, des hydrocolloïdes, des mouillants ou un mélange d'au moins deux des adjuvants susmentionnés.
